(19) Europäisches Patentamt
European Patent Office
Office européen des brevets

(11) EP 3 360 532 B1

(12) # EUROPEAN PATENT SPECIFICATION

(45) Date of publication and mention of the grant of the patent:
**27.04.2022 Bulletin 2022/17**

(21) Application number: **17155913.1**

(22) Date of filing: **13.02.2017**

(51) International Patent Classification (IPC):
***A61K 6/20*** *(2020.01)* ***A61K 6/90*** *(2020.01)*
***C08L 33/10*** *(2006.01)*

(52) Cooperative Patent Classification (CPC):
(C-Sets available)
**A61K 6/20** (Cont.)

(54) **DENTAL COMPOSITION**

DENTALE ZUSAMMENSETZUNG

COMPOSITION DENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.08.2018 Bulletin 2018/33**

(73) Proprietor: **Dentsply DeTrey GmbH**
**78467 Konstanz (DE)**

(72) Inventors:
- **Klee, Joachim**
  **78315 Radolfzell (DE)**
- **Wörner, Melissa**
  **78462 Konstanz (DE)**
- **Szillat, Florian**
  **78462 Konstanz (DE)**
- **Ritter, Helmut**
  **42111 Wuppertal (DE)**

(74) Representative: **Pichova, Vanda**
**Sirona Dental Systems GmbH**
**Corporate Legal**
**Fabrikstraße 31**
**64625 Bensheim (DE)**

(56) References cited:
**EP-A1- 0 020 945** **EP-A2- 0 137 329**
**CN-B- 103 709 257** **DE-A1- 2 343 622**
**DE-A1- 2 552 634** **DE-A1-102010 046 697**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 6/90**

## Description

### Field of the Invention

The present invention relates to a dental impression material containing a polymerizable composition containing a compound having repeating units which are reactive in a radical polymerization. Finally, the present invention relates to a use of a specific polymerizable macromonomer in a dental composition.

The dental composition may be a dental impression material, a flowable dental composite, a universal dental composite, a packable dental composite or a pit and fissure sealer, which compositions are suitable in particular for patients suffering from (meth)acrylate sensitization.

### Background of the Invention

Acrylic monomers such as methyl methacrylate (MMA), triethyleneglycol dimethacrylate (TEGDMA), ethyleneglycol dimethacrylate (EGDMA), 2,2-bis[4-(2-hydroxy-3-methacryloxypropoxy) phenyl]propane (bis-GMA), and 2-hydroxyethyl methacrylate (2-HEMA), are contained in many dental compositions. Dental personnel or patients may become sensitized by exposure to acrylic monomers. Allergic reactions may result when the sensitized personnel or patient becomes exposed again to such allergens. Numerous studies confirm high incidence of sensitization to (meth) acrylates in dental professionals, as well as in patients undergoing dental treatment and exposed to resin-based materials (K. Aalto-Korte et al. Contact Dermatitis 57(5):324-30 · December 2007). Accordingly, exposure to acrylic monomers should be reduced or even completely avoided, notably by individuals suffering from (meth)acrylate sensitization.

On the other hand, dental acrylic preparations provide desirable properties and cannot be easily substituted in dental restorative materials such as dental composites. Therefore, a need exists for dental compositions which do not contain acrylic allergens and which maintain or improve the properties available by the use of (meth)acrylates.

A dental impression is a negative record of the tissues of the mouth which may be used for the fabrication of dentures, crowns or other prostheses. A dental impression is carried out by placing a fluid material in a customized tray into the mouth of the patient. The material then sets to become an elastic solid, and when removed from the mouth retains the shape of the teeth. Conventionally, a dental impression material needs to be activated by mixing and kneading two kinds of pastes which need to be stored separately.

Dental impression materials may be classified on the basis of hydrophilic/hydrophobic properties, the setting mechanism and elasticity. Impression material may set by a reversible physical process or an irreversible chemical reaction for providing rigid or elastic impressions. Considering irreversible chemical reactions, elastic impressions may be obtained by curing of alginate, polysulfide, polyether or silicone impression material whereas rigid impressions are formed by impression plaster or zinc eugenol cement.

The preparation of a dental impression by an irreversible chemical reaction requires the material to have an acceptable working time and setting time. Therefore, conventional dental impression materials are formulated so as to provide working and setting times in a predetermined range. Conventional dental impression materials do not allow to have an arbitrarily long working time while allowing having at the same time a very short setting time.

Document DE 10 2010 046 697 A1 discloses a polymerizable dental material comprising at least one paste component A and at least one paste component B, component A comprising at least one initiator of free-radical polymerization c) selected from the group of the barbituric acid derivatives and/or the malonylsulphamides, and component B comprising at least one organic compound d) comprising acrylic ester and/or methacrylic ester radicals, at least one metal compound e) and at least one halide and/or pseudohalide compound f). Herein, component A, as a reactive paste former, comprises at least one organic compound a) derived from maleic acid and/or from fumaric acid and/or from itaconic acid, and/or at least one compound comprising at least one allyl and/or methallyl radical b).

On the other hand, (meth)acrylate based materials tend to stick to the dental surfaces so that the removal of a cured dental impression from the patient's mouths may be difficult. Moreover, given the large volume of dental impression material, the use of acrylic monomers in a dental impression material poses the risk of sensitization and is undesirable in patients suffering from (meth)acrylate sensitization.

### Summary of the Invention

It is the problem of the present invention to provide a dental impression material which does not give rise to allergic reactions in dental personnel or patients, and which has an arbitrarily long working time and which has at the same time a short setting time, and which provides superior properties of a cured dental composition including mechanical properties, refractive index, and viscosity even in the absence of solvents.

Furthermore, it is the problem of the present invention to provide a polymerizable composition which does not give rise to allergic reactions in dental personnel or patients, and which may be used for the preparation of a dental

impression material or a dental composite for providing superior properties including mechanical properties, refractive index, and viscosity even in the absence of solvents.

Furthermore, it is the problem of the present invention to provide specific polymerizable macromonomers which does not give rise to allergic reactions in dental personnel or patients, for use in a dental impression material or a dental composite.

This problem is solved according to the present invention by a dental impression material comprising

(i) a polymerizable composition containing a compound having repeating units of the following formula (I):

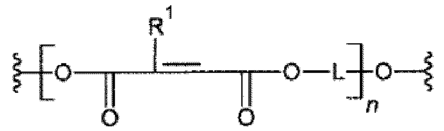

(I)

wherein

$R^1$ represents a hydrogen atom or a linear $C_{1-6}$, or branched or cyclic $C_{3-6}$ alkyl group;
L represents a divalent organic linker group which contains one or more monovalent or divalent organopolysiloxane groups and/or one or more monovalent or divalent perfluorohydrocarbyl groups; and
n is an integer of at least 1;

(ii) one or more compounds copolymerizable with the compound having repeating units of formula (I); and
(iii) a polymerization initiator system.

Finally, the present invention provides a use of a polymerizable macromonomer according to the present invention in a dental composition such as a dental impression material or a dental composite material.

The present invention is based on the recognition that a dental impression material comprising a polymerizable composition containing a compound having repeating units of formula (I) does not give rise to allergic reactions in dental personnel or patients and may be adapted to provide an arbitrarily long working time and a quick setting time and also provides superior properties of the cured composition including mechanical properties, refractive index, and viscosity even in the absence of solvents.

Moreover, the present invention is based on the recognition that a compound having repeating units of the following formula (I) may be cured according to a radical copolymerisation with compounds having polymerizable groups selected from a vinyl group, a vinylether group, and a vinylester group. Furthermore, a compound having repeating units of formula (I) may be cured by a combination of a free-radical copolymerisation with compounds having polymerizable groups selected from a vinyl group, a vinylether group, and a vinylester group, and a cationic polymerisation with a compound having polymerizable groups selected from an aziridine group, an epoxide group and an oxetane group. Accordingly, the dental impression material of the present invention may be adapted to form an interpenetrating network (IPN) by a combination of a free radical polymerization and a cationic polymerization. Therefore, the dental impression material according to the present invention may be used as an alternative for (meth)acrylate based dental compositions for use as a dental impression material, a flowable dental composite, a universal dental composite, a packable dental composite or a pit and fissure sealer.

## Detailed Description of Preferred Embodiments

The linear $C_{1-6}$, or branched or cyclic $C_{3-6}$ alkyl group as defined herein may have the following meaning.

A linear $C_{1-6}$ alkyl group may be selected from methyl, ethan-1-yl, propan-1-yl, butan-1-yl, pentan-1-yl or hexan-1-yl.

A branched $C_{3-6}$ alkyl group may be selected from isopropyl, isobutyl, tert.-butyl, pentan-2-yl, pentan-3-yl, 2-methylbutan-1-yl, 2-methylbutan-2-yl, 2-methylbutan-3-yl, 2,2-dimethylpropyl, hexan-2-yl, hexan-3-yl, 2-methylpentan-1-yl, 3-methylpentan-1-yl, 4-methylpentan-1-yl, 2-methylpentan-2-yl, 3-methylpentan-2-yl, 4-methylpentan-2-yl, 2-methylpentan-3-yl, 3-methylpentan-3-yl, 4-methylpentan-3-yl, 2,2-dimethylbutan-1-yl, 2,3-dimethylbutan-1-yl, 3,3-dimethylbutan-1-yl, 2,2-dimethylbutan-2-yl, 2,3-dimethylbutan-2-yl, and 3,3-dimethylbutan-2-yl.

A cyclic $C_{3-6}$ alkyl group may be selected from a cyclopropyl group, a cyclobutyl group, a cyclopentyl group or a cyclohexyl group.

The term "polymerization" relates to the formation of larger molecules, namely macromolecules or polymers by combining a number of compounds. The term "polymerizable" in the context of a compound refers to the capability of the compound to polymerize under formation of covalent bonds. Polymerizable compounds may form linear macromolecules or they may be combined to form crosslinked polymers having a three-dimensional network structure. According to a particular embodiment, the polymerizable compounds used according to the present invention may form interpenetrating polymer networks (IPNs) by independent polymerization processes. An interpenetrating polymer network is a polymer comprising two or more networks which are at least partially interlaced on a molecular scale but not covalently bonded to each other and cannot be separated unless chemical bonds are broken. Polymerizable compounds having a single polymerizable functional group form linear polymers, whereas polymerizable compounds having at least two polymerizable functional groups may form crosslinked polymers also known as polymer networks. Semi-interpenetrating polymer network (SIPN) may also be formed by forming a polymer comprising one or more networks and one or more linear or branched polymer(s) characterized by the penetration on a molecular scale of at least one of the networks by at least some of the linear or branched macromolecules. Semi-interpenetrating polymer networks are polymer blends since the constituent linear or branched polymers can, in principle, be separated from the constituent polymer network(s) without breaking chemical bonds. Sequential interpenetrating polymer networks are interpenetrating polymer networks prepared by a process in which the second component network is formed following the formation of the first component network. Sequential semi-interpenetrating polymer network are semi-interpenetrating polymer networks prepared by a process in which the linear or branched components are formed following the completion of the reactions that lead to the formation of the network(s) or vice versa.

The term "polymerizable composition" as used herein means a composition containing one or more compounds having at least one polymerizable double bond, preferably a carbon-carbon double bond.

The term "curing" means the polymerization of polymerizable compounds such as monomers, oligomers or even polymers, into preferably a crosslinked polymer network.

The term "polymerization initiator system" as used herein means any compound or mixture of compounds capable of initiating polymerization of polymerizable compounds. The term "polymerization initiator" means any chemical compound forming free radicals when activated, e. g. by exposure to heat, light or interaction with a coinitiator in a photochemical or thermal process. A "photoinitiator system" is a polymerization initiator system which may be activated by light such as actinic light having a wavelength of from 250 to 800 nm.

The present invention provides a polymerizable composition which is polymerizable or copolymerizable by a polymerization initiator system. The polymerizable composition is contained in a dental impression material.

The dental impression material of the present invention comprises a polymerizable composition containing a compound having repeating units of the following formula (I):

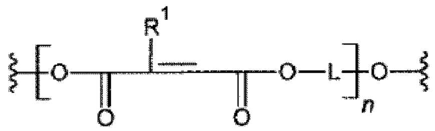

(I)

Formula (I) includes any geometric isomer in view of the carbon-carbon double bond. Accordingly, a compound having repeating units of formula (I) may contain E-isomers or Z-isomers or a mixture of E- and Z-isomers. Preferably, predominantly nonlinear Z-isomers are present in view of the viscosity of the polymerizable composition.

In formula (I1), $R^1$ represents a hydrogen atom or a linear $C_{1-6}$, branched or cyclic $C_{3-6}$ alkyl group. According to a preferred embodiment, $R^1$ is a hydrogen atom.

In formula (I), L represents a divalent organic linker group. The divalent organic linker group may be a divalent hydrocarbyl group. The divalent organic linker group may be derived from an organic diol compound, preferably from an organic di-primary diol compound of the following formula (Ia), which forms a diester linkage in a compound of formula (I):

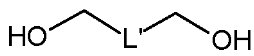

(Ia)

wherein L' is a divalent organic group. The organic group may be selected from a divalent hydrocarbyl group, an organopolysiloxane group and a perfluoroalkylene group.

According to a preferred embodiment, L or L' is a divalent substituted or unsubstituted aliphatic group having 2 to 20 carbon atoms or a divalent aromatic group having 6 to 20 carbon atoms. The divalent aliphatic group having 2 to 20 carbon atoms or the aromatic group having 6 to 20 carbon atoms may have a substituent instead of a hydrogen atom. The divalent aliphatic group here refers to alkylene, substituted alkylene, alkenylene, substituted alkenylene, alkynylene, substituted alkynylene or polyalkyleneoxy. Alkylene, substituted alkylene, alkenylene, and substituted alkenylene are preferred, more preferably alkylene and substituted alkylene. The divalent aliphatic group preferably has a chain structure, more preferably a straight-chain structure.

The divalent aliphatic group preferably contains 2 to 20, more preferably 2 to 15, even more preferably 2 to 12, especially preferably 2 to 10 carbon atoms.

Examples of substituents on the divalent aliphatic group include fluorine atoms, hydroxy, carboxy, amino, aryl, alkoxy, aryloxy, monoalkylamino and dialkylamino.

The divalent aromatic group refers to a divalent monocyclic or polycyclic aromatic hydrocarbon group. Specific examples of the divalent aromatic group include, for example, 1,2-phenylene, 1,3-phenylene, 1,4-phenylene, biphenyl-4,4'-diyl, diphenylmethane-4,4'-diyl, 3,3'-dimethylbiphenyl-4,4'-diyl, 1,2-naphthalene, 1,5-naphthalene, and 2,6-naphthalene. Examples of substituents on the divalent aromatic group include the examples of substituents on the divalent aliphatic group listed above, as well as alkyl.

The divalent organic linker group L may contain one or more monovalent or divalent organopolysiloxane groups. According to a specific embodiment, the divalent organic linker group L contains one monovalent or divalent organopolysiloxane group. According to a further specific embodiment, the divalent organic linker group L contains 2 to 6, preferably 2 to 4 monovalent or divalent organopolysiloxane groups.

A monovalent organopolysiloxane group may be present as a substituent of the divalent hydrocarbyl group or as an end-capping group, and preferably is a group of the following formula (Ib):

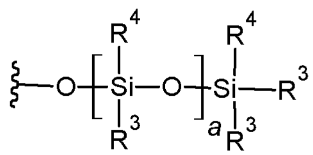

(Ib)

wherein

- $R^3$ which may be the same or different, independently represents a linear $C_{1-6}$, or a branched or cyclic $C_{3-6}$ alkyl group group,
- $R^4$ which may be the same or different when more than one $R^4$ is present, independently represents a vinyl group, a vinylether group, a vinylester group, an aziridine group, an oxetane group, or represents a linear $C_{1-6}$, or a branched or cyclic $C_{3-6}$ alkyl group, or a further group of the formula (Ib), and

wherein a is an integer of from 0 to 50.

A divalent organopolysiloxane group may be a group of the following formula (Ib):

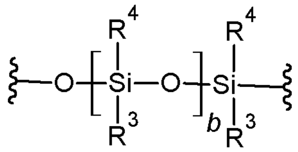

(Ic)

wherein

- $R^3$ which may be the same or different when more than one $R^3$ is present, independently represents a linear $C_{1-6}$, or a branched or cyclic $C_{3-6}$ alkyl group,
- $R^4$ which may be the same or different when more than one $R^4$ is present, independently represents a vinyl group, a vinylether group, a vinylester group, an aziridine group, an oxetane group, or represents a linear $C_{1-6}$, or a branched or cyclic $C_{3-6}$ alkyl group, or a further monovalent group of the formula (Ib), and

wherein b is an integer of from 0 to 50.

Preferably, a polysiloxane group may be selected from polydialkylsiloxanes such as polydimethylsiloxane, polyethylmethylsiloxane or polydiethylsiloxane.

The divalent organopolysiloxane group may bridge two fragments of the divalent organic linker group or form a cyclic structure with a portion of the divalent organic linker group. According to a preferred embodiment, L is a divalent hydrocarbyl group which may contain one or more monovalent or divalent polysiloxane groups.

The divalent organic linker group may also contain one or more monovalent or divalent perfluorohydrocarbyl groups. The perfluorohydrocarbyl group is a hydrocarbyl group of which all hydrogen atoms are substituted by fluorine atoms.

According to a preferred embodiment, L is a divalent hydrocarbyl group which may contain one or more monovalent or divalent perfluorohydrocarbyl groups. The monovalent perfluorohydrocarbyl groups include alkyl and aryl perfluorocarbons; suitable perfluorohydrocarbyl groups are, for example, trifluoromethyl, pentafluoroethyl, pentafluorophenyl, and heptafluoronaphthyl. The aryl groups of the hydrocarbyl groups are preferably $C_{1-18}$ alkyl groups or $C_{6-20}$ aryl or aralkyl groups.

Preferably, L is a polyalkylene group or a polyalkyleneoxide group, which groups may contain a monovalent or divalent polysiloxane groups, or L is a divalent group containing a combination of a polyalkylene group, a polyalkyleneoxide group, and a polysiloxane group.

A polyalkylene group may be a C2-20 polyalkylene group, more preferably, a C2-12 alkylene group such as an ethylene group, a propylene group, a butylene group, a pentylene group, a hexylene group, a heptylene group, an octylene group, a nonylene group, a decylene group, an undecylene group or a dodecylene group.

A polyalkylene oxide group may be a poly C2-6 alkylene oxide group such as a polyethylene oxide group, a polypropylene oxide group, a polybutylene oxide group a polypentylene oxide group or a polyhexylene oxide group. Preferably, the poly C2-6 alkylene oxide group has 2 to 20 repeating units.

A divalent group containing a combination of a polyalkylene group, a polyalkyleneoxide group, and polysiloxane group maybe a group of the following formula (Id):

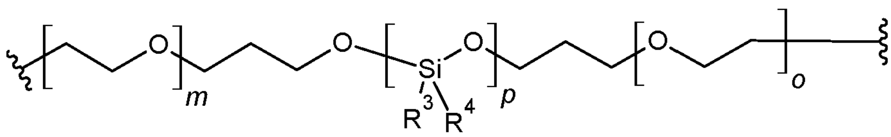

(Id)

wherein

- m is an integer of from 0 to 20,
- o is an integer of from 0 to 20,
- p is an integer of from 1 to 40,
- $R^3$ represents a linear $C_{1-6}$, or a branched or cyclic $C_{3-6}$ alkyl group group,
- $R^4$ represents a vinyl group, a vinyl ether group, a vinyl ester group, an aziridine group, an oxetane group or represents a linear $C_{1-6}$, or a branched or cyclic $C_{3-6}$ alkyl group group,

According to a preferred embodiment, L is a divalent organic linker group containing a divalent organopolysiloxane group. Preferably, the organopolysiloxane group contains a polymerizable group selected from vinylether, vinylester, oxetane, and aziridine groups.

In formula (I), n is an integer of at least 1. The integer n is preferably in the range of from 1 to 70. More preferably, n is in the range of from 2 to 20.

The polymerizable composition containing a compound having repeating units of formula (I) is obtainable by reacting a mixture comprising:

(a) x equivalents of one or more compounds of the following formula (I):

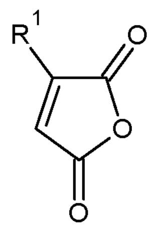

(II)

wherein
$R^1$ is as defined above;
(b) y equivalents of a compound of the following formula (II):

HO-L-OH (III)

wherein
L is as defined above; and
(c) optionally z equivalents of one or more compounds of the following formula(IV) or (V):

$R^2$—COOH

(IV)

$R^2$—OH

(V)

wherein

$R^2$ is an organic group which may contain one or more monovalent or divalent organopolysiloxane groups and/or one or more monovalent or divalent perfluorohydrocarbyl groups, and/or one or more polymerizable groups selected from (meth)acryloyl groups, (meth)acrylamido groups, vinylether groups, vinylester groups,
wherein $0.05 \leq x/y \leq 0.66$, and $2y - 2x \leq z \leq 1.5(2y - 2x)$, wherein x, y, and z are the molar equivalents of components (a), (b) and (c).

$R^2$ is an organic group which may contain one or more monovalent or divalent organopolysiloxane groups and/or one or more monovalent or divalent perfluorohydrocarbyl groups, and/or one or more polymerizable groups selected from (meth)acryloyl groups, (meth)acrylamido groups, vinylether groups, vinylester groups. According to a preferred embodiment, $R^2$ is an aromatic group. When $R^2$ is an aromatic group, the refractive index of the dental impression material tends to increase. Preferably, the dental impression material according to the present invention contains a polymerizable composition (i) having a refractive index of from 1.450 to 1.540.

The dental impression material according to the present invention further one or more compounds copolymerizable with the compound having repeating units of formula (I). The one or more compounds copolymerizable with the compound having repeating units of formula (I) are selected from vinyl compounds. The vinyl compounds may be vinyl ethers and derivatives thereof, vinyl esters and derivatives thereof, vinylsiloxanes and derivatives thereof, as well as (meth)acrylates and (meth) acrylamides. Specific examples of vinyl ethers and derivatives thereof are selected from

divinyl ether, ethylene glycol divinyl ether, diethylene glycol divinyl ether, triethyleneglycol divinyl ether, tetraethylene glycol divinyl ether, vinyl-2-chloroethyl ether, vinyl-n-butyl ether, 1,4-cyclohexanedimethanol divinyl ether, 1,4-cyclohexanediol divinyl ether, vinylglycidyl ether, bis(4-(vinyloxymethyl)cyclohexylmethyl)glutarate, tri(ethyleneglycol) divinyl ether, 1,4-butanediol divinyl ether, tetramethylene glycol divinyl ether, neopentyl glycol divinyl ether, trimethylol propane trivinyl ether, trimethylol ethane trivinyl ether, bis(4-(vinyloxy) butylisophthalate, hexanediol divinyl ether, pentaerythritol divinyl etherdivinyl ether, allylvinyl ether, methylvinyl ether, ethylvinyl and ether.

Vinyl esters and derivatives thereof, are obtainable by the esterification of an epoxy resin with an unsaturated monocarboxylic acid, and include vinyl acetate, vinyl benzoate, vinyl cinnamate, vinyl 4-tert-butylbenzoate, vinyl stearate, vinyl propionate, vinyl valerate, vinyl pivalate vinyl decanoate, divinyl ester allyl vinyl ester, adipic acid divinyl ester, tris(4-vinyloxy)butyl trimellilate, bis(4-(vinyloxy)butyl) terephthalate, tetraethylene glycol divinyl ether, pentaerythritol trivinyl ether, and butenyl vinyl ester.

Vinylsiloxanes and derivatives thereof may be selected from vinylpolysiloxanes and polyvinylpolysiloxanes such as monovinyldimethyl terminated polydimethylpolysiloxane.

(Meth)acrylates may be present in reduced amounts and can be selected from methyl (meth)acrylate, isobutyl (meth)acrylate, benzyl (meth)acrylate, lauryl (meth)acrylate, 2-(N,N-dimethylamino)ethyl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, N-methylol (meth)acrylamide, N-hydroxyethyl (meth)acrylamide, N-(dihydroxyethyl)(meth)acrylamide, (meth)acryloyloxydodecylpyridinium bromide, (meth)acryloyloxydodecylpyridinium chloride, (meth)acryloyloxyhexadeylpyridinium chloride, (meth)acryloyloxydecylammonium chloride, ethylene glycol di(meth)acrylate, triethylene glycol di(meth)acrylate, propylene glycol di(meth)acrylate, neopentyl glycol di(meth)acylate, 1,6-hexanediol di(meth)acrylate, 1,10-decanediol di(meth)acrylate, bisphenol A diglycidyl (meth)acrylate (2,2-bis[4-[3-(meth)acryloyloxy-2-hydroxypropoxy]phenyl]propane, commonly known as "BisGMA"), 2,2-bis[4-(meth)acryloyloxyethoxyphenyl]propane, 2,2-bis[4-(meth)acryloyloxyolyothoxyphenyl]propane, 2,2-bis[4-[3-((meth)acryloyloxy-2-hydroxypropoxy]phenyl]propane, 1,2-bis[3-(meth)acryloyloxy-2-hydroxypropoxylethane, pentaerythritol di(meth)acrylate, [2,2,4-trimethylhexamethylenebis(2-carbamoyloxyethyl)] dimethacrylate (commonly known as "UDMA trimethylolpropane tri(meth)acrylate, trimethylolethane tri(meth)acrylate, tetramethylolmethane tri(meth)acrylate, pentaerythritol tetra(meth)acrylate, dipentaerythritol hexa(meth)acrylate, N,N'-(2,2,4-trimethylhexamethylene)bis[2-(aminocarbonyloxy)propane-1,3-diol] tetramethacrylate, and 1,7-diacryloyloxy-2,2,6,6-tetraacryloyloxymethyl-4-oxyheptane.

(Meth) acrylamides may be have the following formulae:

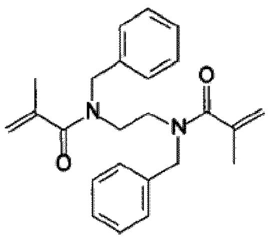 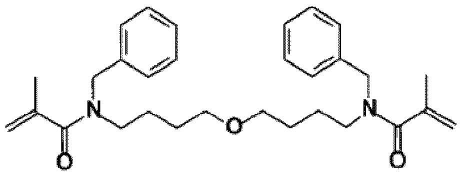 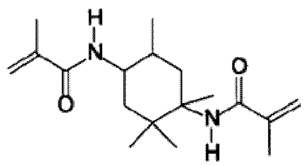

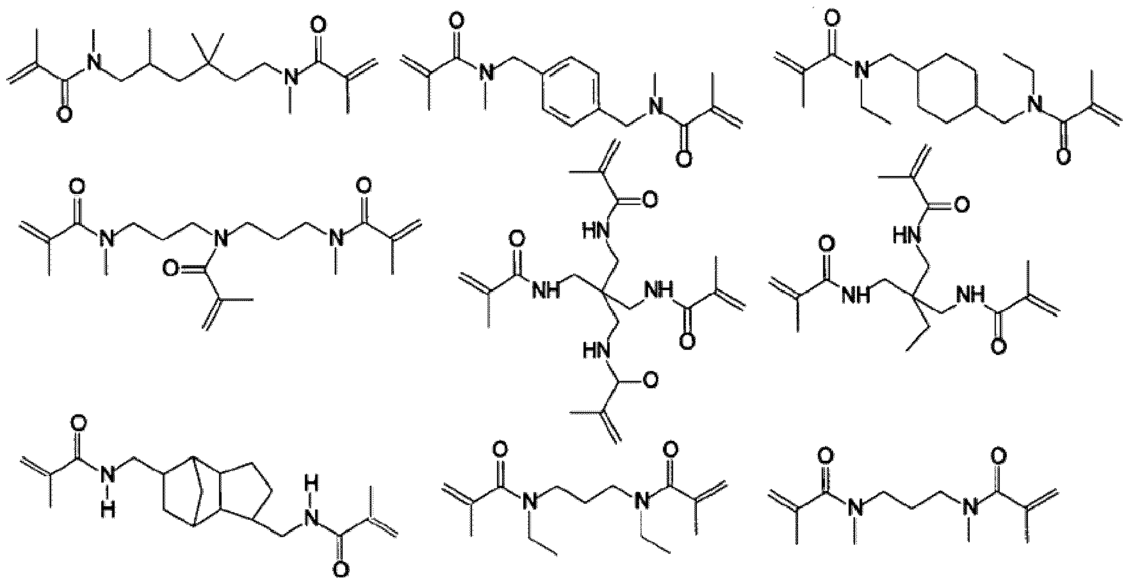

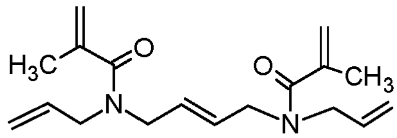

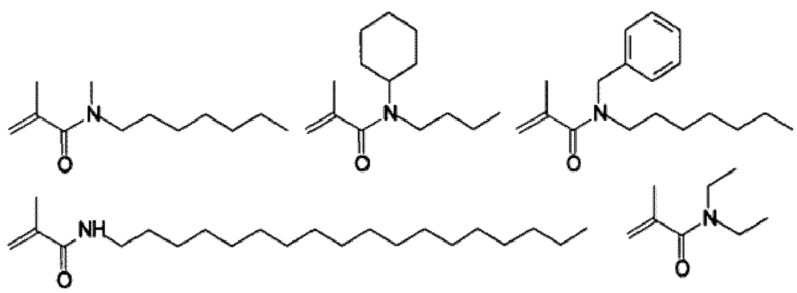

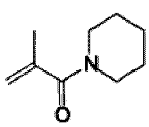

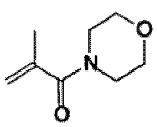

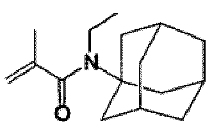

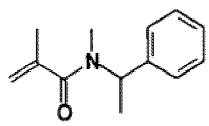

Preferred acrylamides according to formulae (D), (E), (F) have the following formulae:

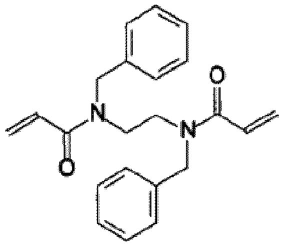

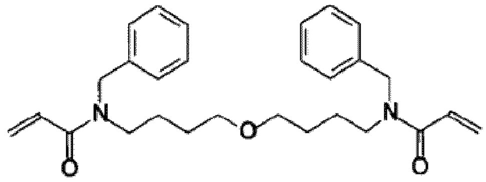

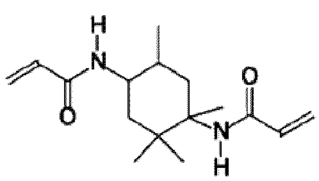

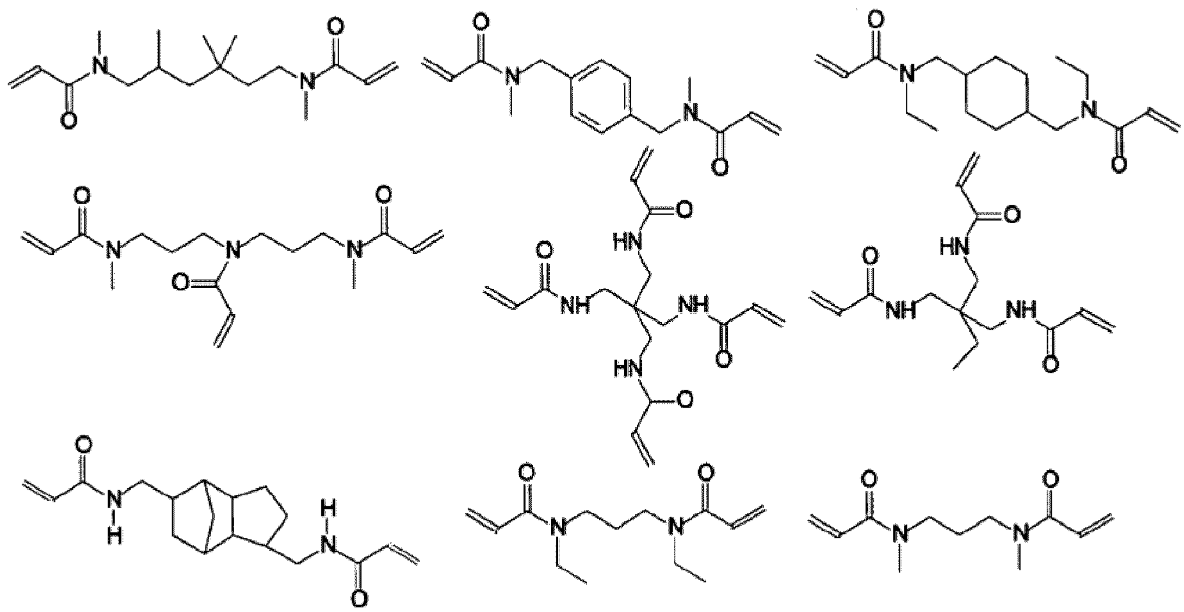

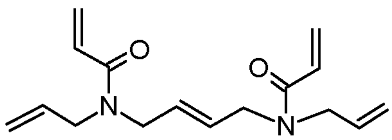

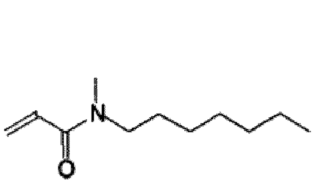 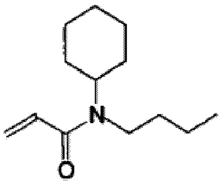 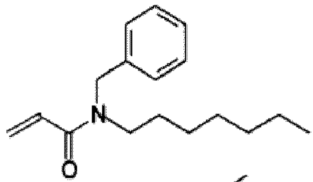

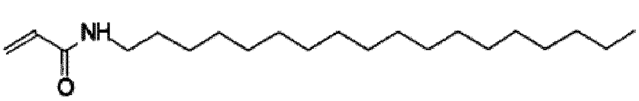 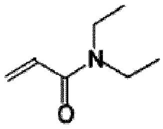

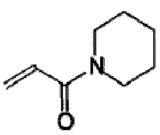 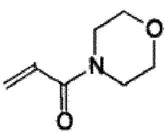 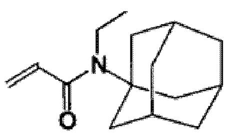 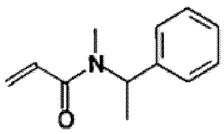 .

Most preferred are the following bisacrylamides:

N,N'-diallyl-1,4- bisacrylamido-(2E)-but-2-en (BAABE) having the structural formula

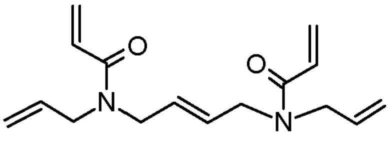 ,

and

N,N'-diethyl-1,3-bisacrylamido-propan (BADEP) having the structural formula

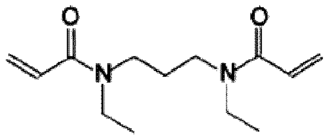

.

The vinyl compounds may also be selected from monofunctional monomers selected from 1-alkenes having from 4 to 20 carbon atoms and include the following: 1-butene; 1-pentene; 1-hexene; 1-heptene; 1-octene; 1-nonene; 1-decene; 1-dodecene; 1-tetradecene; 1-hexadecene; 1-heptadecene; 1-octadecene; 2-methyl-1-butene; 3,3-dimethyl-1-pentene; 2-methyl-1-heptene; 4,4-dimethyl-1-heptene; 3,3-dimethyl-1-hexene; 4-methyl-1-pentene; 1-eicosene; 1-docosene; 1-tetracosene; 1-hexacosene; 1-octacosene; 1-triacontene; 1-dotriacontene; 1-tetratriacontene; 1-hexatriacontene; 1-octatriacontene; 1-tetracontene; 1-dotetracontene; 1-tetratetracontene; 1-hexatetracontene; 1-octatetracontene; 1-pentacontene; 1-hexacontene and mixtures thereof.

The dental impression material according to the present invention further contains (iii) a polymerization initiator system. The polymerization initiator system (iii) may be any compound or system capable of initiating the polymerization of the polymerizable composition containing a compound having repeating units of formula (I).

A polymerization initiator system may be a binary or ternary system. A binary system may include a polymerization initiator and an electron donor compound, and a ternary system may further include a component capable of catalysing radical and/or cationic polymerization, in particular in case the composition contains cationically polymerizable groups such as a vinyl group, a vinylether group, a vinylester group, an aziridine group, an oxetane group. Examples of suitable catalysts are selected from an iodonium salt, a sulfonium salt, and a phosphonium salt, as for example described in US 5,545,676.

Suitable photoinitiators for the polymerization initiator system (ii) are Norrish type I and Norrish type II photoinitiators.

Suitable Norrish type I photoinitiators are phosphine oxides and Si- or Ge-acyl compounds.

Phosphine oxide photoinitiators may have a functional wavelength range of about 380 nm to about 450 nm, which include acyl and bisacyl phosphine oxides such as those described in US 4,298,738, US 4,324,744 US and 4,385,109 and EP 0 173 567. Specific examples of the acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphonate, and 2,4,6-trimethylbenzoylethoxyphenylphosphine oxide. Commercially available phosphine oxide photoinitiators capable of free-radical initiation when irradiated at wavelength ranges of greater than about 380 nm to about 450 nm include bis(2,4,6-trimethylbenzoyl)phenyl phosphine oxide (IRGACURE 819), bis(2,6-dimethoxybenzoyl)-(2,4,4-trimethylpentyl) phosphine oxide (CGI 403), a 25:75 mixture, by weight, of bis(2,6-dimethoxybenzoyl)-2,4,4-trimethylpentyl phosphine oxide and 2-hydroxy-2-methyl-1-phenylpropan-1-one (IRGACURE 1700), a 1:1 mixture, by weight, of bis(2,4,6-trimethylbenzoyl)phenyl phosphine oxide and 2-hydroxy-2-methyl-1-phenylpropane-1-one (DAROCUR 4265), and ethyl 2,4,6-trimethylbenzylphenyl phosphinate (LUCIRIN LR8893X). Typically, the phosphine oxide initiator is present in the composition in catalytically effective amounts, such as from 0.1 percent by weight to 5.0 percent by weight, based on the total weight of the composition.

Si- or Ge-acyl compound photoinitiators preferably have the following formula (X):

$$X^P\text{-}R^P \qquad (X)$$

wherein

$X^P$ is a group of the following formula (XI):

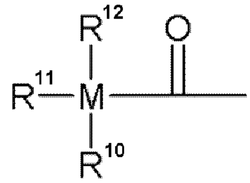

(XI)

wherein

M is Si or Ge;
$R^{10}$ represents a substituted or unsubstituted hydrocarbyl or hydrocarbylcarbonyl group;
$R^{11}$ represents a substituted or unsubstituted hydrocarbyl or hydrocarbylcarbonyl group;
$R^{12}$ represents a substituted or unsubstituted hydrocarbyl group; and
$R^P$ (i) has the same meaning as $X^P$, whereby the compound of formula (X) may be symmetrical or unsymmetrical; or (ii) is a group of the following formula (XII):

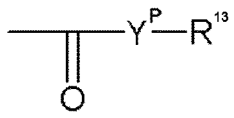

(XII)

wherein
$Y^P$ represents a single bond, an oxygen atom or a group NR', wherein R' represents a substituted or unsubstituted hydrocarbyl group;
$R^{13}$ represents a substituted or unsubstituted hydrocarbyl group, a trihydrocarbylsilyl group, a mono(hydrocarbylcarbonyl)dihydrocarbylsilyl group or a di(hydrocarbylcarbonyl)monohydrocarbylsilyl group; or
(iii) when M is Si, $R^P$ may be a substituted or unsubstituted hydrocarbyl group.

Photoinitiator compounds of formula (X) represent polymerization initiators which are particularly suitable for dental impression materials. Compounds of formula (X) provide a high polymerization efficiency, and coloration problems are avoided. Moreover, in a polymerization system comprising a conventional photoinitiator such as camphor quinone, coloration is efficiently suppressed. Furthermore, compounds of formula (X) have a light absorption within the wavelength range typically applied in dental application, they are compatible with the ingredients of dental impression materials and besides, they are considered physiologically harmless. Therefore, compounds of formula (X) are particularly preferred as photoinitiators.

In connection with compound of formula (X), the term "substituted" as used herein means that $R^{10}$, $R^{11}$, $R^{12}$, $R^{13}$ and R' may be substituted by a substituent selected from the group consisting of halogen atoms, a nitro group, a cyano group, a hydroxy group, an amino group, $C_{1-6}$ alkyl groups, $C_{1-6}$ alkoxy groups and a $-NR^xR^y$ group wherein $R^x$ and $R^y$ independently from each other represent a $C_{1-6}$ alkyl group. Here, illustrative of the halogen atoms can be fluorine, chlorine, bromine and iodine. The $C_{1-6}$ alkyl groups are, for example, methyl, ethyl, n-propyl, isopropyl and n-butyl. Illustrative of the $C_{1-6}$ alkoxy groups are, for example, methoxy, ethoxy and propoxy. The alkyl moieties in these substituents may be linear, branched or cyclic. Preferably, the substituent is selected from a chlorine atom, a nitro group, a $C_{1-4}$ alkoxy group and a $-NR^xR^y$ group wherein $R^x$ and $R^y$ independently from each other represent a $C_{1-4}$ alkyl group.

If $R^{10}$, $R^{11}$ and $R^{12}$ are substituted, then it is preferred that they are substituted with 1 to 3 substituents, more preferably with 1 substituent.

In the compound of formula (X), moieties $R^{10}$, $R^{11}$ and $R^{12}$ may be defined as follows:
$R^{10}$ and $R^{11}$ independently from each other represent a substituted or unsubstituted hydrocarbyl or hydrocarbylcarbonyl group, and $R^{12}$ represents a substituted or unsubstituted hydrocarbyl group.

The hydrocarbyl group may be an alkyl group, a cycloalkyl group, a cycloalkylalkyl group, an arylalkyl group or an aryl group.

An alkyl group may be straight-chain or branched $C_{1-20}$ alkyl group, typically a $C_{1-8}$ alkyl group. Examples for a $C_{1-6}$ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl.

A cycloalkyl group may be a $C_{3-20}$ cycloalkyl group, typically a $C_{3-8}$ cycloalkyl group. Examples of the cycloalkyl group can include those having 3 to 6 carbon atoms, for example, cyclopropyl, cyclobutyl, cyclopentyl and cyclohexyl.

A cycloalkylalkyl group may have 4 to 20 carbon atoms and may include a combination of a linear or branched alkyl group having 1 to 6 carbon atoms and a cycloalkyl group having 3 to 14 carbon atoms. Examples of the cycloalkylalkyl(-) group can for example, include methylcyclopropyl(-) methylcyclobutyl(-), methylcyclopentyl(-), methylcyclohexyl(-), ethylcyclopropyl(-), ethylcyclobutyl(-), ethylcyclopentyl(-), ethylcyclohexyl(-), propylcyclopropyl(-), propylcyclobutyl(-), propylcyclopentyl(-), propylcyclohexyl(-).

An arylalkyl(-) group may be a $C_{7-20}$ arylalkyl(-) group, typically a combination of a linear or branched alkyl group having 1 to 6 carbon atoms and an aryl(-) group having 6 to 10 carbon atoms. Specific examples of an arylalkyl(-) group are a benzyl(-) group or a phenylethyl(-) group.

An aryl group can include aryl groups having 6 to 10 carbon atoms. Examples of the aryl group are phenyl and

naphtyl.

The hydrocarbylcarbonyl groups of $R^{10}$ and $R^{11}$ represent acyl groups ($R_{org}$-(C=O)-) in which the organic residue $R_{org}$ is a hydrocarbyl residue as defined above.

Compound of formula (X) may contain one or two hydrocarbylcarbonyl groups, that is either one of $R^{10}$ or $R^{11}$ is a hydrocarbylcarbonyl group, or both $R^{10}$ and $R^{11}$ are hydrocarbylcarbonyl groups. Preferably, compound of formula (X) contains one hydrocarbylcarbonyl group.

Preferably, the hydrocarbylcarbonyl group is an arylcarbonyl group, more preferably a benzoyl group.

Preferably, $R^{10}$ and $R^{11}$ are independently selected from the group consisting of a straight chain or branched $C_{1-6}$ alkyl group, and a phenyl or benzoyl group which may optionally be substituted by one to three substituents selected from halogen atoms, a nitro group, a $C_{1-4}$ alkoxy group and a -$NR^xR^y$ group wherein $R^x$ and $R^y$ independently from each other represent a $C_{1-4}$ alkyl group, and $R^{12}$ is a straight chain or branched $C_{1-6}$ alkyl group or a phenyl group.

Most preferably, $R^{10}$ and $R^{11}$ are independently selected from the group consisting of a straight chain or branched $C_{1-4}$ alkyl group, and a phenyl or benzoyl group which may optionally be substituted with one substituent selected from the group consisting of selected from a halogen atom, a nitro group, a $C_{1-4}$ alkoxy group and a -$NR^xR^y$ group wherein $R^x$ and $R^y$ independently from each other represent a $C_{1-4}$ alkyl group, and $R^{12}$ is a straight chain or branched $C_{1-4}$ alkyl group.

In the compound of formula (X), $R^P$ may have the same meaning as $X^P$, whereby the compound of formula (X) may be symmetrical or unsymmetrical. Alternatively, $R^P$ may represent a substituted or unsubstituted hydrocarbyl group, or a group of formula (XII). Preferably, if $R^P$ has the same meaning as $X^P$, then compound of formula (X) is unsymmetrical. If $R^P$ represents a substituted or unsubstituted hydrocarbyl group, then the hydrocarbyl group has the same meaning as defined above for $R^6$ and is independently selected therefrom.

In the group of formula (XII) of compound of formula (X), $R^{13}$ represents a substituted or unsubstituted hydrocarbyl group, a trihydrocarbylsilyl group, a mono(hydrocarbylcarbonyl)-dihydrocarbylsilyl group or a di(hydrocarbylcarbonyl)monohydrocarbylsilyl group.

If $R^{13}$ of formula (XII) is a trihydrocarbylsilylgroup, a mono(hydrocarbylcarbonyl)-dihydrocarbylsilyl group or a di(hydrocarbylcarbonyl)monohydrocarbylsilyl group, each of the hydrocarbyl and hydrocarbylcarbonyl groups has the same meaning as defined for $R^{10}$, $R^{11}$ and $R^{12}$ and is independently selected therefrom.

In formula (XII), R' has the same meaning as defined for $R^{12}$ and is independently selected therefrom.

If M is Si in compound of formula (X), $R^P$ may be also be a substituted or unsubstituted hydrocarbyl group, wherein the hydrocarbyl group has the same meaning as defined above for $R^{12}$ and is independently selected therefrom.

For example, compounds of formula (X) wherein $R^P$ has the same meaning as $X^P$ and which are symmetrical may be have the following structural formulae:

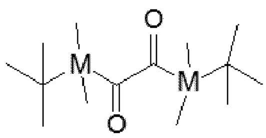 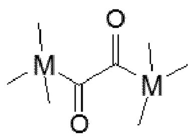.

For example, compounds of formula (X) wherein $R^P$ represents a group of formula (XII) wherein $Y^P$ is a bond, an oxygen atom or a NR' group, and $R^{13}$ represents a substituted or unsubstituted hydrocarbyl group may have the following structural formulae:

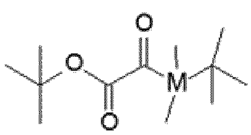 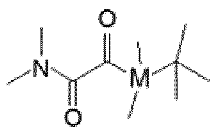 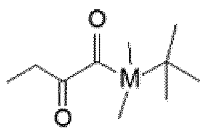 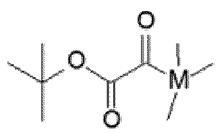

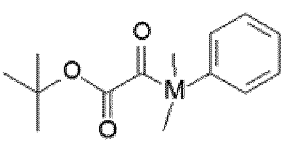 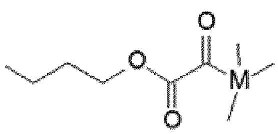 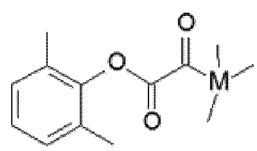

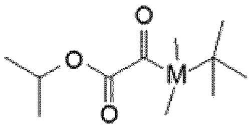
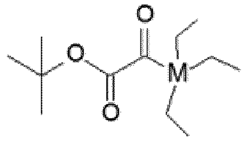
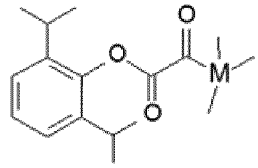

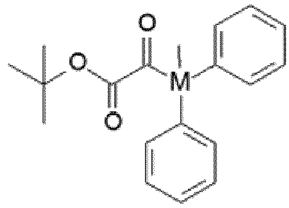
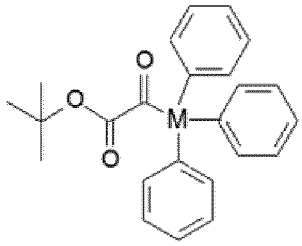

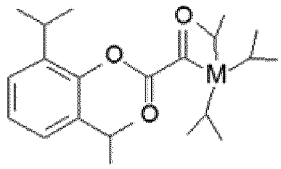
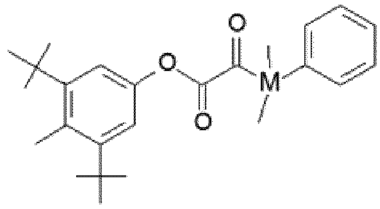

For example, compounds of formula (X) wherein $R^P$ represents a group of formula (XII) wherein $R^{13}$ represents a trihydrocarbylsilyl group have the following structural formulae:

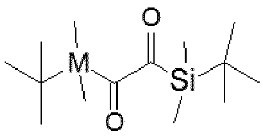
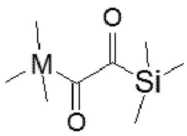

For example, compounds of formula (X) wherein M is Si and $R^P$ represents a substituted or unsubstituted hydrocarbyl group, may have the following structural formulae:

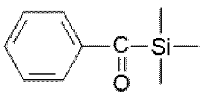
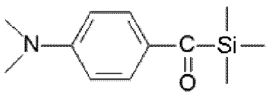
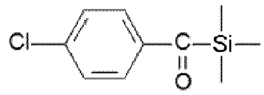

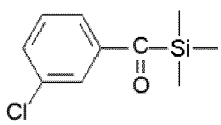
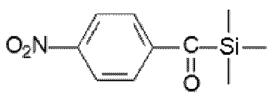
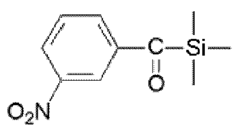

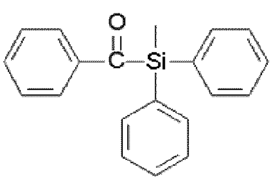
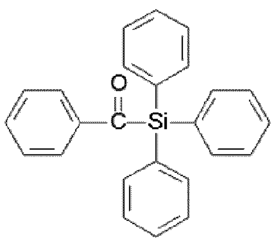
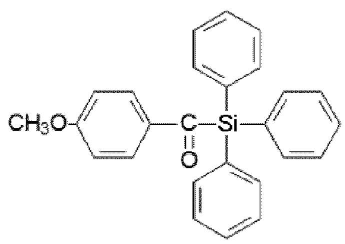

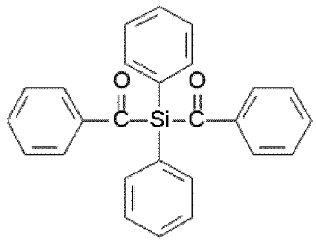 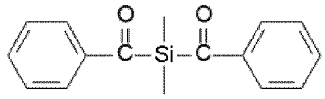 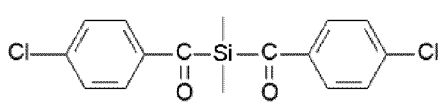

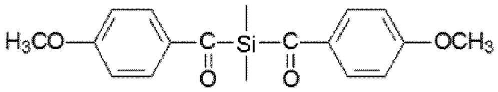 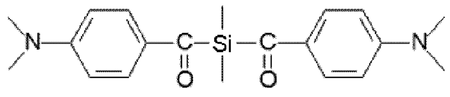

Preferably, compound of formula (X) is selected from the group consisting of:

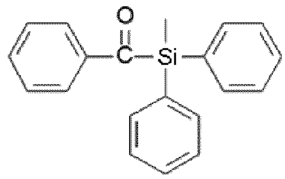 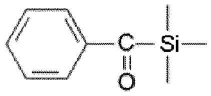 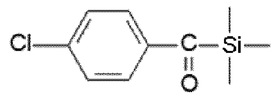 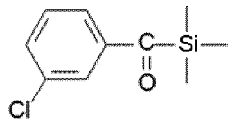

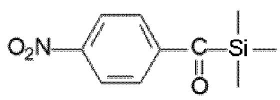 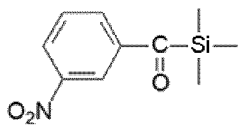 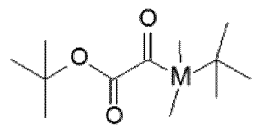 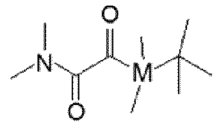

,

wherein compounds of formula (X) with M = Si are particularly preferred.

Most preferably, compound of formula (X) is selected from the group consisting of: compound of formula (X) is selected from the group consisting of:

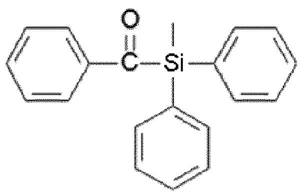 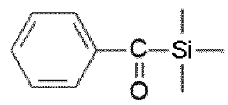 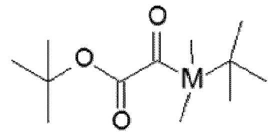

,

wherein it is particularly preferred that M = Si.

Suitable Norrish type II photoinitiators are for example combinations of sensitizer compounds such as monoketones and diketones that absorb some light within a range of about 250 nm to about 520 nm (preferably, about 450 nm to about 500 nm) with a coinitiator. Particularly suitable sensitizer compounds include alpha diketones that have some light absorption within a range of about 250 nm to about 520 nm (even more preferably, about 450 to about 500 nm). Examples of sensitizer compounds include camphor quinone, benzil, furil, 3,3,6,6-tetramethylcyclo-hexanedione, phenanthraquinone, 1-phenyl-1,2-propanedione and other 1-aryl-2-alkyl-1,2-ethanediones, and cyclic alpha diketones. Suitable coinitiators are electron donor compounds including substituted amines, e.g., ethyl dimethylaminobenzoate or dimethylamino benzonitrile, or a polymerizable compound having CH-acidity such as the present polymerizable compounds of formula (IV).

Tertiary amine reducing agents may be used in combination with an acylphosphine oxide Examples of suitable aromatic tertiary amine include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, 4-N,N-dimethylaminobenzoic acid ethyl ester, 4-N,N-dimethylaminobenzoic acid methyl ester, 4-N,N-dimethylaminobenzoic acid n-butoxyethyl ester, 4-N,N-dimethylaminobenzoic acid 2-(methacryloyloxy) ethyl ester, 4-N,N-dimethylaminobenzophenone ethyl 4-(N,N-dimethylamino)benzoate and N,N-dimethylaminoethyl methacrylate. Examples of an aliphatic tertiary amine include trimethylamine, triethylamine, N-methyldiethanolamine, N-ethyldiethanolamine, N-n-butyldiethanolamine, N-lauryldiethanolamine, triethanolamine, 2-(dimethylamino) ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

The amine reducing agent may be present in the composition in an amount from 0.1 percent by weight to 5.0 percent by weight, based on the total weight of the composition.

In case the dental impression material is in the form of an acidic composition, that is a composition having a pH of less than 7, depending on the composition's pH level, it is preferred to select compounds of formula (X) with the proviso that they do not contain ester groups, or at least only ester groups which do not significantly hydrolyse in aqueous media at pH 3 at room temperature within one month. Thereby, an advantageous stability of an acidic dental impression material, that is a composition having a pH of less than 7, in terms of shelf-life stability of the uncured dental impression material as well as stability after curing in the mouth of a patient is ensured. Therefore, for acidic dental impression materials, particularly preferred are compounds of formula (X) excluding $R^P$ being a group of formula (XII) in which $Y^P$ is an oxygen atom.

Furthermore, since the acylsilyl moiety (-C(=O)-Si-) might be sensitive to basic conditions, that is a pH higher than 7, it is preferred to suitably select a pH value of the composition being higher than 7 with the proviso that the acylsilyl moiety is not cleaved in aqueous media at the selected basic pH at room temperature within one month.

The compound of the formula (X) may be a known compound which is commercially available or a may be prepared according to published procedures.

The compound of formula (X) wherein M is Si and $R^P$ represents a substituted or unsubstituted hydrocarbyl group may for example be readily prepared by means of a one-step Pd-catalyzed reaction with a disilane as described e.g. by Yamamoto K. et al., J. Tetrahedron Lett., 1980, vol. 21, pages 1653 to 1656:

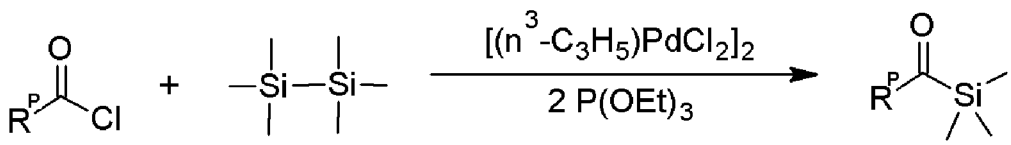


Scheme 3: Preparation of acylsilanes

In Scheme 3, the reaction is exemplary depicted with hexamethylsilan as the disilane, whereby a compound of formula (X) wherein $R^{10}$, $R^{11}$ and $R^{12}$ represent a methyl group is obtained. It is understood that $R^{10}$, $R^{11}$ and $R^{12}$ can be varied by applying disilanes having hydrocarbon substituents other than methyl.

The compound of formula (X) wherein $R^P$ represents a group of formula (XII) in which $Y^P$ is an oxygen atom and $R^{13}$ represents a hydrocarbyl group may for example be prepared by a three-step synthesis as described by Nicewicz D.A. et al. in Org. Synth., 2008, 85, pages 278 to 286. In this three-step synthesis, an acetoacetate is converted to an azide compound, which is then reacted with a trihydrocarbylsilyltrifluoromethane-sulfonate to obtain a trihydrocarbylsilyldiazoacetate, which is finally reacted with potassium peroxymonosulfate to arrive at the target compound:

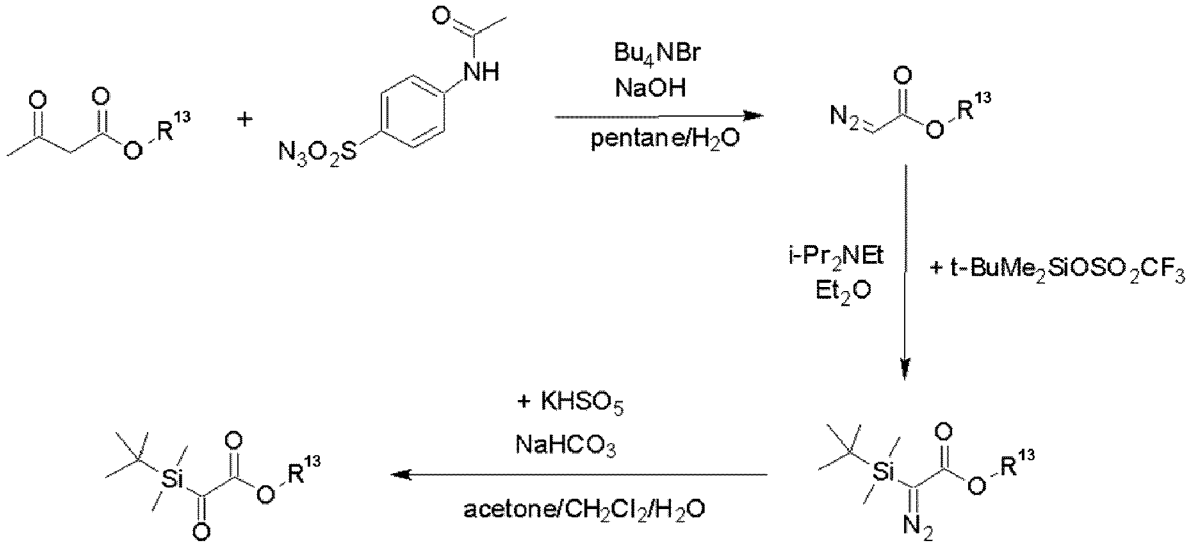


Scheme 4: Preparation of silylglyoxylates

In Scheme 4, the reaction is exemplary depicted for obtaining a compound of formula (X) wherein in $X^P$ of formula (X), $R^{10}$ and $R^{11}$ represent a methyl group, and $R^{12}$ represents a tert-butyl group. It is understood that $R^{10}$, $R^{11}$ and $R^{12}$ can be varied by applying a trihydrocarbylsilyltrifluoromethane-sulfonate other than t-BuMeSiOSO$_2$CF$_3$.

Alternatively, compounds of formula (X) wherein M is Si, $R^P$ represents a group of formula (XII) and $Y^P$ represents an oxygen atom may be prepared by a single-pot three-component coupling reaction of a silylglyoxylate, a terminal alkyne and an aldehyde in the presence of $ZnI_2$ and $Et_3N$ as described by Nicewicz D.A. in J. Am. Chem. Soc., 2005, 127 (17), pages 6170 to 6171. Further syntheses of silylglyoxylate compounds are described e.g. by Boyce G.R. et al. in J. Org. Chem., 2012, 77 (10), pages 4503 to 4515 and Boyce G.R. et al. in Org. Lett., 2012, 14 (2), pages 652 to 655.

For example, the following compounds of formula (X) are known and commercially available, and their Chemical Abstracts (CAS) No. is given in brackets: benzoyltriphenylsilane (1171-49-9), benzoyltrimethylsilan (5908-41-8), 1-[(trimethylsilyl) carbonyl]-naphthalene (88313-80-8), 1-methoxy-2-[(trimethylsilyl)-carbonyl]- benzene (107325-71-3), (4-chlorobenzoyl) (triphenyl) silane (1172-90-3), (4-nitrobenzoyl) (triphenyl) silane (1176-24-5), (methyldiphenylsilyl)phenylmethanone (18666-54-1), (4-methoxybenzoyl) triphenylsilan (1174-56-7) and tert-butyl (tert-butyldimethylsilyl)glyoxylate (852447-17-7).

All compounds of formula (X) comprise the group of formula (XI)

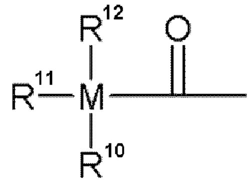

(XI).

In formula (XI), M, $R^{10}$, $R^{11}$ and $R^{12}$ are defined as above. Depending on the selection of M, the group of formula (XI) represents an acylsilane or acylgermane group. Upon exposure to UV-VIS-light, the bond between M and the acyl group may be cleaved, whereby a silyl/germanyl and an acyl radical is formed as a polymerization initiating structure, but in competition to the cleavage into to radicals, a carbene structure might be formed:

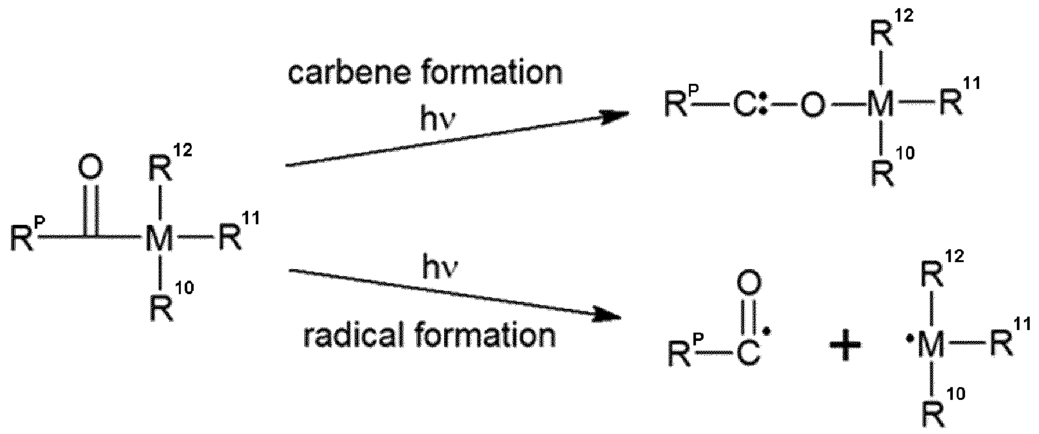


Scheme 5: Carbene formation versus radical formation

This competition between the formation of polymerization initiating radicals and carbene formation is described for acylsilanes by El-Roz, M. et al. in Current Trends in Polymer Science, 2011, vol. 15, pages 1 to 13.

Besides, in case in compound of formula (X) wherein $R^P$ has the same meaning as $X^P$ or is a group of formula (XII), the C-C bond of the 1,2-diketone moiety (-C(=O)-C(=O)-) may be cleaved upon exposure to UV-VIS-light into two acyl radicals. This cleavage is exemplary shown for compound of formula (X) wherein $R^P$ is a group of formula (XII) and $Y^P$ is an oxygen atom, that is for a glyoxylate (-O-C=O)-C(=O)-) compound:

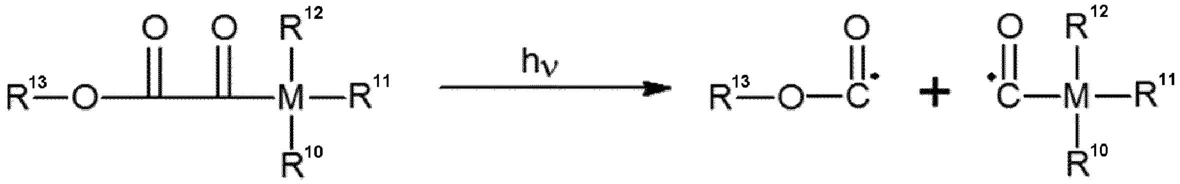


Scheme 6: Cleavage of -O-C(=O)-C(=O)- moiety of a glyoxylate

Besides, in compound of formula (X), there is a third possibility for a radical cleavage in case $R^P$ is a compound of formula (XII) wherein $Y^P$ is an oxygen atom and $R^{13}$ is a substituted or unsubstituted hydrocarbyl group. Namely, an intra- or intermolecular hydrogen abstraction might occur, where a hydrogen radical is abstracted:

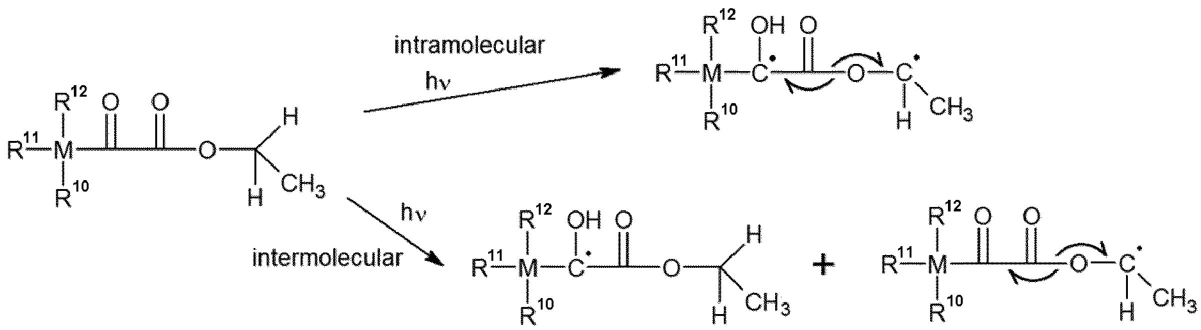


Scheme 7: Hydrogen abstraction (intra- or intermolecular)

Both the cleavage of a glyoxylate group and the hydrogen abstraction mechanism is known for photoinitiators which do not contain silicium or germanium, such as ethyl phenylglyoxylate (Irgacure® MBF).

For compounds of formula (X) wherein $R^P$ has the same meaning as $X^P$ or is a group of formula (XII), the present inventors carried out molecular modelling calculations from which it appears that a Si-C or Ge-C bond cleavage can be ruled out, since the C-C bond of the -C(=O)-C(=O)- moiety is weaker than the Si-C or Ge-C bond.

The photoinitiator system may further comprise diaryl iodonium salts, triaryl sulfonium salts and tetraaryl or tetraalkyl phosphonium salts. These salts may serve as a coinitiator for improving the polymerization performance of

the photoinitiator, but they may also serve as an initiator for cationic polymerization.

For example, diaryl iodonium salt may be selected from the group consisting of (4-methylphenyl)[4-(2-methylpropyl) phenyl] iodonium hexafluoroantimonate, include (4-methylphenyl)[4-(2-methylpropyl) phenyl] iodonium tetrafluoroborate, diphenyliodonium (DPI) tetrafluoroborate, di(4-methylphenyl)iodonium (Me2-DPI) tetrafluoroborate, phenyl-4-methylphenyliodonium tetrafluoroborate, di(4-heptylphenyl)iodonium tetrafluoroborate, di(3-nitrophenyl)iodonium hexafluorophosphate, di(4-chlorophenyl)iodonium hexafluorophosphate, di(naphthyl)iodonium tetrafluoroborate, di(4-trifluoromethylphenyl)iodonium tetrafluoroborate, DPI hexafluorophosphate, Me2-DPI hexafluorophosphate; DPI hexafluoroarsenate, di(4-phenoxyphenyl)iodonium tetrafluoroborat, phenyl-2-thienyliodonium hexafluorophosphate, 3,5-dimethylpyrazolyl-4-phenyliodonium hexafluorophosphate, DPI hexafluoroantimonate, 2,2'-DPI tetrafluoroborate, di(2,4-dichlorophenyl)iodonium hexafluorophosphate, di(4-bromophenyl)iodonium hexafluorophosphate, di(4-methoxyphenyl)iodonium hexafluorophosphate, di(3-carboxyphenyl)iodonium hexafluorophosphate, di(3-methoxycarbonylphenyl)iodonium hexafluorophosphate, di(3-methoxysulfonylphenyl)iodonium hexafluorophosphate, di(4-acetamidophenyl)iodonium hexafluorophosphate, di(2-benzothienyl)iodonium hexafluorophosphate, and DPI hexafluorophosphate.

Particularly preferred iodonium compounds include diphenyliodonium (DPI) hexafluorophosphate, di(4-methylphenyl)iodonium (Me2-DPI) hexafluorophosphate, diaryliodonium hexafluoroantimonate, (4-methylphenyl)[4-(2-methylpropyl) phenyl] iodonium hexafluoroantimonate, (4-methylphenyl)[4-(2-methylpropyl)phenyl]iodonium hexafluorophosphate (Irgacure® 250, commercial product available from BASF SE), (4-methylphenyl)[4-(2-methylpropyl) phenyl] iodonium tetrafluoroborate, 4-octyloxyphenyl phenyliodonium hexafluoroantimonate, 4-(2-hydroxytetradecyloxyphenyl)phenyliodonium hexafluoroantimonate, and 4-isopropyl-4'-methyldiphenyliodonium borate.

According to a particularly preferred embodiment, the iodonium compound is DPI hexafluorophosphate and/or 4-isopropyl-4'-methyldiphenyliodonium tetrakis(pentafluorophenyl) borate.

A preferred triaryl sulfonium salt is S-(phenyl)thianthrenium hexafluorophosphate of the following formula:

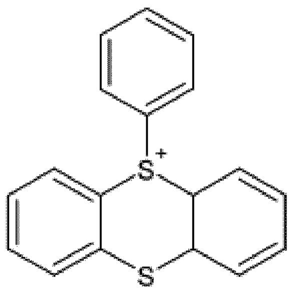

$PF_6$.

Particularly preferred phosphonium salts are the tetraalkyl phosphonium salts tetrakis-(hydroxymethyl)-phosphonium (THP) salt or a tetrakis-(hydroxymethyl)-phosphonium hydroxide (THPOH) salt, wherein the anion of the tetraalkyl phosphonium salt is selected from the group consisting of formate, acetate, phosphate, sulphate, fluoride, chloride, bromide and iodide.

A particularly preferred photoinitiator system comprises a photoinitiators of formula (X), optionally in addition with camphor quinone, in combination with a diaryl iodonium salt, triaryl sulfonium salt or a tetraaryl or tetraalkyl phosphonium salt as described above.

Preferably, the polymerization initiator is contained in an amount of from 0.01 to 10 percent based on the total weight of the composition. The amount of the polymerization initiator to be added in the present invention is not particularly limited. Preferably, 0.01 to 10 parts by weight of the polymerization initiator per 100 parts by weight of the polymerizable composition may be used. When the amount of the polymerization initiator is less than 0.01 part by weight, polymerization may not proceed sufficiently and thereby mechanical strength may be reduced. Therefore, the amount is more preferably at least 0.1 part by weight. On the other hand, when the amount of the polymerization initiator exceeds 10 parts by weight, in the case where the polymerization initiator itself has low polymerization performance, sufficient mechanical strength may not be obtained and furthermore precipitation from the composition may occur.

The dental composite of the present invention may further contain a polymerization accelerator. Examples of the polymerization accelerator are amines and sulfinic acids and salts thereof.

Preferably, the polymerizable composition comprises polymerizable macromonomer of the following formula (VI):

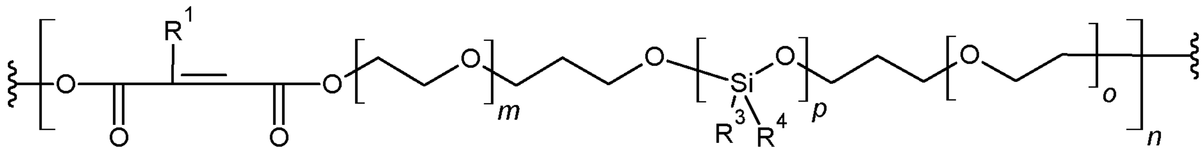

(VI)

wherein

- m is an integer of from 0 to 20,
- o is an integer of from 0 to 20,
- p is an integer of from 1 to 40,
- $R^1$ represents a hydrogen atom or a linear $C_{1-6,}$ branched $C_{2-6}$ or cyclic $C_{3-6}$ alkyl group,
- $R^3$ represents a linear $C_{1-6,}$ branched $C_{2-6}$ or cyclic $C_{3-6}$ alkyl group,
- $R^4$ represents a vinyl group, an aziridine group, an oxetane group or a linear $C_{1-6,}$ branched $C_{2-6}$ or cyclic $C_{3-6}$ alkyl group, and

wherein n is an average chain length which is in the range of from 1 to 50.

The polymerizable macromonomer of the formula (VI) may be used in a dental composition. Preferably, the dental composition is a dental impression material, a flowable dental composite, a universal dental composite, a packable dental composite or a pit and fissure sealer.

The total amount of the polymerizable monomers contained in the dental impression material or the dental composite besides the polymerizable composition containing macromers is preferably in the range of from 1 part to 100 part by weight per 100 part by weight of the reaction product of components (a), (b), and (c) of the polymerizable composition.

The dental impression material preferably further comprises a filler. The filler is an ingredient for adjusting the viscosity and flowability of the dental impression material before curing and the strength after curing. A filler may be included in a range of from 0.1 to 2 part by weight per 1 part by weight of the curable matrix. Illustrative examples of the filler include reinforcing fillers such as calcium carbonate, wet finely powdered silica, fumed silica, crystalline silica, carbon black, red iron oxide, cerium oxide, titanium oxide, and aluminum hydroxide; and fillers obtained by hydrophobizing the surface of these fillers with an organosilicon compound.

The dental composite of the present invention may further contain a pH adjuster, an ultraviolet absorber, an antioxidant, a polymerization inhibitor, a colorant, an antibacterial agent, an X-ray contrast agent, a thickening agent, a fluorescent agent.

The dental composite of the present invention may further contain a fluorine ion sustained-releasable filler, such as sodium fluoride, calcium fluoride, fluoroaluminosilicate glass, or sodium monofluorophosphate.

The dental composite may contain an antimicrobial agent. The antimicrobial agent may be a surfactant having an antibacterial activity, such as 12-(meth)acryloyloxydodecylpyridinium bromide or cetylpyridinium chloride.

The present invention will now be further illustrated based on the following examples.

## Examples

Materials: Maleic anhydride (purity 95% or 99 %), 1,6-hexanediol (HEX, purity 97% or 99%), 1,10-decanediol (DEC, purity 98%), 2,2-dimethylpropane-1,3-diol (NPG, purity 99%), monoethyl fumarate (purity 95 %), 2-methyl-1,3-propanediol (purity 99%), $Bi(OTf)_3$, 1,4-butanediol divinyl ether (BDDVE, purity 98%), triethylenglycol divinyl ether (TEGDVE, purity 98%), 1,3-bis(4-hydroxybutyl)tetramethyldisiloxane ( purity >97%) and 2,2,3,3,4,4,5,5,6,6,7,7,8,8,9,9-hexadecafluoro-1,10-decanediol (purity 97%) were purchased from Sigma-Aldrich.

Butylated hydroxytoluene was purchased from F. B. SILBERMANN GmbH & Co. KG.

Camphorquinone and ethyl 4-(dimethylamino)benzoate (DMABE) were purchased from PCM Products GmbH.

Divinyl adipate (DVA; purity > 99%) was purchased from TCI Deutschland GmbH.

## Synthesis of UP-Resins

### Preparation Example 1 (MEW-01-003-02), comparative

In a 100 mL round bottom flask within a Tornado™ Overhead Stirring System from Radley's, 10 g (0.1020 mol) of maleic anhydride and 12.40 g (0.1049 mol) of 1,6-hexanediol were heated up to 180°C (heating bath temperature).

The mixture was stirred for 6 h at 180°C and 300 mbar. After cooling down, the remaining water was removed in vacuum at 40°C. A yellowish, clear liquid was obtained. The IR spectrum is shown in Fig. 1.

$$\eta = 38.1\ Pa*s$$

$$IR\ (cm^{-1}): 1717\ (ester\ C{=}O)$$

**Preparation Example 2 (MEW-01-026-01), comparative**

In a 100 mL round bottom flask with a magnetic stir bar and a distillation bridge to remove the produced water, 10 g (0.1020 mol) of maleic anhydride and 18.168 g (0.1042 mol) of 1,10-decanediol were heated to 180°C (heating bath temperature). The reaction mixture was stirred for 6 h at 180°C. After cooling down, the remaining water was removed in vacuum at 40°C. A colorless, clear liquid was obtained which became cloudy after some days.

$$\eta = 8.6\ Pa*s$$

$$IR\ (cm^{-1}): 1720\ (ester\ C{=}O)$$

**Preparation Example 3 (MEW-01-112-01), comparative**

In a three neck flask with a mechanical stirrer, a gastight magnetic stirrer head and a distillation bridge, 40.404 g (0.4079 mol) of maleic anhydride, 45.172 g (0.4294 mol) of 2,2-dimethylpropane-1,3-diol and 89.9 mg (0.4079 mmol) of butylated hydroxytoluene were heated to 160°C (heating bath temperature). After 1 h of stirring at 160°C, a vacuum of 950 mbar was set. After another hour, 179.8 mg (0.8158 mmol) of butylated hydroxytoluene were added and the reaction mixture was stirred for 13 h at 160°C and 950 mbar.

$$\eta = 85.7\ Pa*s$$

Conversion of 2,2-dimethylpropane-1,3-diol according to NMR: 76.3%
$^1H$ NMR ($CDCl_3$, 400 MHz) δ (ppm) = 6.92 - 6.80 (m, HC=CH fumarate, 0.38H), 6.44 - 6.28 (m, HC=CH-COOH, 0.71H), 6.26 (s, HC=CH maleate, 0.69H), 4.09 - 3.95 (m, $CH_2O$, 3.02H), 3.52 (s, $CH_2OH$ starting material, 0.20H), 3.40 - 3.32 (m, $CH_2OH$ chain end, 0.74H), 1.01 (dt, $C(\underline{CH_3})_2$, 6H)

**Preparation Example 4 (MEW-01-110-01), inventive**

In a 50 mL round bottom flask with a mechanical stirrer, 916.3 mg (9.25 mmol) of maleic anhydride, 4.639 g (9.74 mmol) of 2,2,3,3,4,4,5,5,6,6,7,7,8,8,9,9-hexadecafluoro-1,10-decanediol and 2.0 mg (0.0093 mmol) of butylated hydroxytoluene were heated to 180°C (heating bath temperature). After 2h of stirring at 180°C, 4.1 mg (0.0185 mmol) of butylated hydroxytoluene were added and the reaction mixture was stirred for another hour at 180°C. After cooling down, an off-white solid was obtained which is soluble in tri(ethylene glycol) divinyl ether.

**Preparation Example 5 (MEW-01-116-01), inventive**

In a 100 mL round bottom flask within a Tornado™ Overhead Stirring System from Radley's, 6.061 g (0.0612 mol) of maleic anhydride, 17.940 g (0.0644 mol) of 1,3-bis(4-hydroxybutyl)tetramethyldisiloxane and 27.0 mg (0.1224 mmol) of butylated hydroxytoluene were heated up to 180°C (heating bath temperature). The mixture was stirred for 2 h at 180°C and a colorless, clear liquid was obtained.

$$\eta = 0.536\ Pa*s$$

Conversion of 1,3-bis(4-hydroxybutyl)tetramethyldisiloxane according to NMR: 72.5%
$^1H$ NMR ($CDCl_3$, 400 MHz) δ (ppm) = 6.85 (s, HC=CH fumarate, 0.25H), 6.49-6.32 (m, HC=CH-COOH, 0.77H),

6.23 (s, HC=CH maleate, 0.80H), 4.32 - 4.25 (m, 77H), 4.18 (t, 2.10H), 3.90-3.86 (m, 0.08H), 3.70-3.62 (m, 1.01H), 1.85-1.80 (m, 0.09H), 1.78-1.65 (m, 2.82H), 1.64-1.55 (m, 1.18H), 1.47-1.33 (m, 3.92H), 0.66 - 0.60 (m, 0.16H), 0.58 - 0.48 (m, 3.80H), 0.20 - 0.11 (m, 0.45H), 0.08 - 0.00 (s, 11.55H)

**Preparation Example 6 (MEW-01-122-01_B), comparative**

In a 100 mL round bottom flask within a Tornado™ Overhead Stirring System from Radley's, 6.8721 g (0.0694 mol) of maleic anhydride, 9.1242 g (0.0867 mol) of 2,2-dimethylpropane-1,3-diol, 5.2632 g (0.0347 mol) of monoethyl fumarate and 22.9 mg (0.1041 mmol) of butylated hydroxytoluene were heated up to 180°C (heating bath temperature). After 1 h of stirring at 180°C, a vacuum of 800 mbar was set with a water jet pump. After another hour, 45.9 mg (0.2081 mmol) of butylated hydroxytoluene were added and the reaction mixture was stirred for 22 h at 180°C and 800 mbar.

Conversion of 2,2-dimethylpropane-1,3-diol according to NMR: 99.2% $^1$H NMR ($CDCl_3$, 400 MHz) δ (ppm) = 6.94 - 6.80 (m, HC=CH fumarate, 1.83H), 6.25 (s, HC=CH maleate, 0.29H), 4.25 (q, $OCH_2CH_3$, 0.29H), 4.11 - 3.95 (m, $OCH_2$, 3.93H), 3.36 (s, $CH_2OH$, 0.03H), 1.31 (t, $OCH_2\underline{CH_3}$, 0.38H), 1.06 - 0.90 (m, $C(\underline{CH_3})_2$, 6H)

**Preparation Example 7 (MEW-01-081-01), comparative**

Synthesis according to: Kricheldorf, H. R.; Yashiro, T.; Weidner, S.; Macromolecules 2009, 42, 6433-6439

In a 250 mL round bottom flask within a Tornado™ Overhead Stirring System from Radley's, 40.404 g (0.4079 mol) of maleic anhydride, 38.1934 (0.4196 mol) of 2-methyl-1,3-propanediol and 1.338 g (2.040 mmol) of $Bi(OTf)_3$ were heated to 80°C. After 1 h of stirring at 80°C, vacuum was increased with a water jet pump within 1 h to 12 mbar and the mixture was stirred for 22 h.

After cooling down, the resin was dissolved in DCM (900 mL) and extracted with 5% NaOH (6 x 100 mL). The combined aqueous phases were washed with DCM and the combined organic phases were washed with water. After drying with $MgSO_4$, 57.72 mg of butylated hydroxytoluene were added and the solvent was evaporated.

$$\eta = 1492\ Pa{*}s$$

Conversion of 2-methyl-1,3-propanediol according to NMR: 92.8%

$^1$H NMR ($CDCl_3$, 400 MHz) δ (ppm) = 6.26 (s, HC=CH maleate, 1.77H), 4.24 -4.00 (m, $OCH_2$, 3.71H), 3.60-3.46 (m, $HOCH_2$, 0.29H), 2.32-2.18 (m, $\underline{CH}CH_2O$, 0.89H), 2.08 -1.98 (m, $\underline{CH}CH_2OH$, 0.16H), 1.07 - 0.90 (m, $CH_3$, 3H)

## Application Examples

### General Procedure of Copolymerisation

The UP-resin was mixed with divinyl ether or divinyl ester. 1 mol % of the amount of double bonds of camphorquinone and 1.3 mol % of the amount of double bonds of DMABE were added. After obtaining a homogenous mixture, all formulations of Table 1 (comparative examples) were cured with the Smart Lite Focus (Dentsply Sirona). Furthermore, photo-DSC (DSC 7b Perkin Elmer) measurements are summarized in Tabl.1.

Table 1: Copolymerization of UP-resin with cross-linker

| *App. Ex.* | *MEW-01-XXX-01* | *Diol* | *Cross-linker* | *Amount DB* [%]* | *T [°C]* | *Exposure time [min]* | *Peak [min]* | *ΔH [J/g]* | *η at 23°C [Pa*s]* |
|---|---|---|---|---|---|---|---|---|---|
| *1* | *025* | *HEX* | *BD DVE* | *100* | *37* | 8.1 | *0.48* | *-26.00* | *1.46* |
| *2* | *031* | *HEX* | *BD DVE* | *100* | *37* | *20.1* | *0.53* | *-45.00* | *5.6* |
| *3* | *035* | *HEX* | *TEG DVE* | *100* | *37* | *20.1* | *0.51* | *-40.83* | 38.75 |
| *4* | *035* | *HEX* | *TEG DVE* | *100* | *80* | *20.1* | *0.46* | *-63.01* | 38.75 |
| *5* | *047* | *DEC* | *TEG DVE* | *100* | *37* | *20.1* | *0.62* | -49.98 | 6.77 |
| *6* | *091_B* | *NPG* | *TEG DVE* | 38 | *37* | *15.1* | *1.13* | -26.87 | 29.2 |

(continued)

| App. Ex. | MEW-01-XXX-01 | Diol | Cross-linker | Amount DB* [%] | T [°C] | Exposure time [min] | Peak [min] | ΔH [J/g] | η at 23°C [Pa*s] |
|---|---|---|---|---|---|---|---|---|---|
| 7 | 091_H | NPG | DVA | 39 | 37 | 15.1 | 0.76 | -24.15 | 22.3 |
| *Amount DB = Amount of double bonds in cross-linker compared to amount of double bonds in the UP-resin | | | | | | | | | |

**Application Example 8 (MEW-01-124-01), inventive**

1 g of an UP-resin with perfluorinated parts (MEW-01-110-01) was dissolved in 0.307 g of triethylenglycol divinyl ether and mixed homogenously with 8 mg of camphorchinone and 9 mg of DMABE. The mixture was cured with a Smart Lite Focus and a brittle solid was obtained.

**Application Example 9 and 10 (MEW-01-125-01), inventive**

The following mixtures were made with an UP-resin that obtains siloxane parts (MEW-01-116-01).

Table 2: Copolymerization of UP-siloxane resin with cross-linker

| ***Application Example*** | *9 (MEW-01-125-01_A)* | *10 (MEW-01-125-01_C)* |
|---|---|---|
| *UP-resin (MEW-01-116-01)* | *4.998 g* | *2.000 g* |
| *TEG DVE* | *1.359 g* | *-* |
| *DVA* | *-* | *0.530 g* |
| *Camphorquinone* | *44 mg* | *18 mg* |
| *DMABE* | *66 mg* | *27 mg* |

The mixtures were cured with a Smart Lite Focus (Dentsply Sirona) within 20s resulting in hard solids.

**Claims**

**1.** A dental impression material comprising

(i) a polymerizable composition containing a compound having repeating units of the following formula (I):

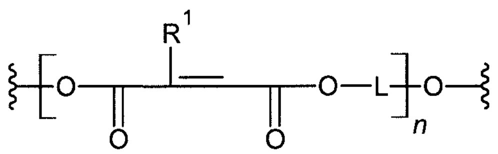

(I)

wherein

$R^1$ represents a hydrogen atom or a linear $C_{1-6}$ alkyl group, or a branched or cyclic $C_{3-6}$ alkyl group;
L represents a divalent organic linker group which contains one or more monovalent or divalent organopolysiloxane groups and/or one or more monovalent or divalent perfluorohydrocarbyl groups; and
n is an integer of at least 1;

(ii) one or more compounds copolymerizable with the compound having repeating units of formula (I); and
(iii) a polymerization initiator system.

**2.** The dental impression material according to claim 1, wherein the polymerizable composition containing a compound having repeating units of formula (I) is obtainable by reacting a mixture comprising:

(a) x equivalents of one or more compounds of the following formula (II):

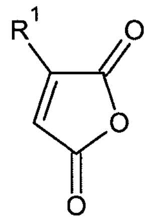

(II)

wherein $R^1$ is as defined in claim 1;
(b) y equivalents of a compound of the following formula (III):

HO-L-OH (III)

wherein
L is as defined in claim 1; and
(c) optionally z equivalents of one or more compounds of the following formula (IV) or (V):

$R^2$-COOH (IV)

$R^2$-OH (V)

wherein

> $R^2$ independently is an organic group which may contain one or more monovalent or divalent organopolysiloxane groups and/or one or more monovalent or divalent perfluorohydrocarbyl groups, and/or one or more polymerizable groups selected from (meth)acryloyl groups, (meth)acrylamido groups, vinyl ether groups, vinyl ester groups, aziridine, epoxide groups and oxetane groups,

wherein $0.05 \leq x/y \leq 0.66$, and $2y - 2x \leq z \leq 1.5(2y - 2x)$, wherein x, y, and z are the molar equivalents of components (a), (b) and (c).

**3.** The dental impression material according to claim 1 or 2, wherein $R^1$ is a hydrogen atom.

**4.** The dental impression material according to any one of the preceding claims, wherein L is a divalent organic linker group containing a divalent organopolysiloxane group.

**5.** The dental impression material according to any one of the preceding claims, wherein the organopolysiloxane group contains a polymerizable group selected from vinylether, vinylester, oxetane, and aziridine groups.

**6.** The dental impression material according to any one of the preceding claims, which is adapted to form an interpenetrating network (IPN) by a combination of a free radical polymerization and a cationic polymerization.

**7.** The dental impression material according to any one of the preceding claims, wherein the polymerizable composition (i) has a refractive index of from 1.450 to 1.540.

**8.** A polymerizable composition comprising polymerizable macromonomers of one of the following formula (VI):

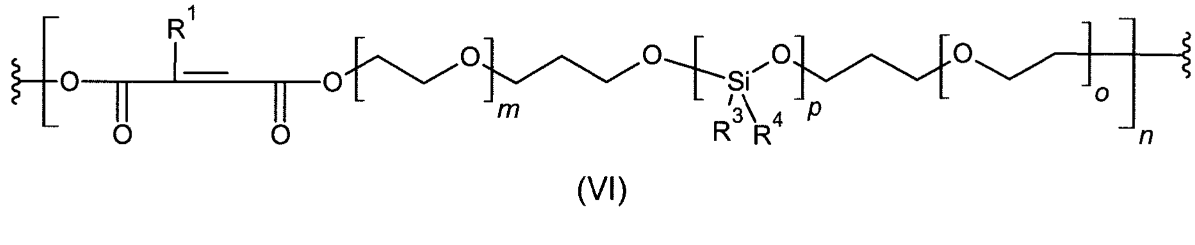

(VI)

wherein

m is an integer of from 0 to 20,
o is an integer of from 0 to 20,
p is an integer of from 1 to 40,
$R^1$ represents a hydrogen atom or a linear $C_{1-6}$, branched $C_{2-6}$ or cyclic $C_{3-6}$ alkyl group,
$R^3$ represents a linear $C_{1-6}$, or a branched or cyclic $C_{3-6}$ alkyl group group,
$R^4$ represents a vinyl group, a vinylether group, a vinylester group, an aziridine group, an oxetane group or a represents a linear $C_{1-6}$, or a branched or cyclic $C_{3-6}$ alkyl group, and

wherein n is an average chain length which is in the range of from 1 to 50.

**9.** Use of a polymerizable macromonomer of the formula (VI) as defined in claim 8 in a dental composition.

**10.** The use according to claim 9, wherein the dental composition is a dental impression material, a flowable dental composite, a universal dental composite, a packable dental composite or a pit and fissure sealer.

**Patentansprüche**

**1.** Dentales Abformmaterial, umfassend

(i) eine polymerisierbare Zusammensetzung, die eine Verbindung mit sich wiederholenden Einheiten der folgenden Formel (I) enthält:

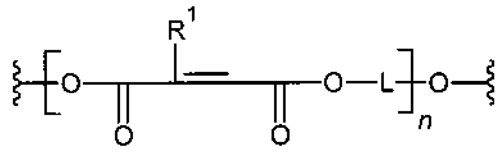

(I)

wobei

$R^1$ ein Wasserstoffatom oder eine lineare $C_{1-6}$-Alkylgruppe oder eine verzweigte oder cyclische $C_{3-6}$-Alkylgruppe darstellt;
L eine zweiwertige organische Linkergruppe darstellt, die eine oder mehrere einwertige oder zweiwertige Organopolysiloxangruppen und/oder eine oder mehrere einwertige oder zweiwertige Perfluorkohlenwasserstoffgruppen enthält; und
n eine ganze Zahl von mindestens 1 ist;

(ii) eine oder mehrere Verbindungen, die mit der Verbindung mit wiederkehrenden Einheiten der Formel (I) copolymerisierbar sind; und
(iii) ein Polymerisationsinitiatorsystem.

**2.** Dentales Abformmaterial nach Anspruch 1, wobei die polymerisierbare Zusammensetzung, die eine Verbindung mit sich wiederholenden Einheiten der Formel (I) enthält, erhältlich ist durch Umsetzen einer Mischung, umfassend:

(a) x Äquivalente einer oder mehrerer Verbindungen der folgenden Formel (II):

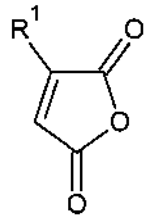

(II)

wobei
$R^1$ wie in Anspruch 1 definiert ist;
(b) y Äquivalente einer Verbindung der folgenden Formel (III):

HO-L-OH (III)

wobei
L wie in Anspruch 1 definiert ist; und
(c) gegebenenfalls z Äquivalente einer oder mehrerer Verbindungen der folgenden Formel (IV) oder (V):

$R^2$-COOH (IV)

$R^2$-OH (V)

wobei

$R^2$ unabhängig eine organische Gruppe ist, die eine oder mehrere einwertige oder zweiwertige Organopolysiloxangruppen und/oder eine oder mehrere einwertige oder zweiwertige Perfluorkohlenwasserstoffgruppen und/oder eine oder mehrere polymerisierbare Gruppen enthalten kann, ausgewählt aus (Meth)acryloylgruppen, (Meth)acrylamidogruppen, Vinylethergruppen, Vinylestergruppen, Aziridin, Epoxidgruppen und Oxetangruppen,

wobei $0{,}05 \leq x/y \leq 0{,}66$ und $2y-2x \leq z \leq 1{,}5(2y-2x)$, wobei x, y und z die molaren Äquivalente der Komponenten (a), (b) und (c) sind.

**3.** Dentales Abformmaterial nach Anspruch 1 oder 2, wobei $R^1$ ein Wasserstoffatom ist.

**4.** Dentales Abformmaterial nach einem der vorhergehenden Ansprüche, wobei L eine zweiwertige organische Linkergruppe ist, die eine zweiwertige Organopolysiloxangruppe enthält.

**5.** Dentales Abformmaterial nach einem der vorhergehenden Ansprüche, wobei die Organopolysiloxangruppe eine polymerisierbare Gruppe enthält, die aus Vinylether-, Vinylester-, Oxetan- und Aziridingruppen ausgewählt ist.

**6.** Dentales Abformmaterial nach einem der vorhergehenden Ansprüche, das ausgelegt ist, um durch eine Kombination aus einer freien radikalischen Polymerisation und einer kationischen Polymerisation ein interpenetrierendes Netzwerk (IPN) zu bilden.

**7.** Dentales Abformmaterial nach einem der vorhergehenden Ansprüche, wobei die polymerisierbare Zusammensetzung (i) einen Brechungsindex von 1,450 bis 1,540 aufweist.

**8.** Polymerisierbare Zusammensetzung, umfassend polymerisierbare Makromonomere einer der folgenden Formel (VI):

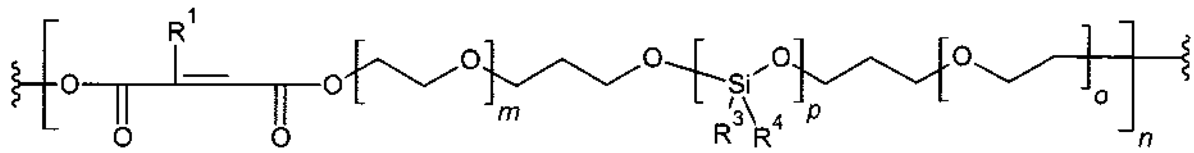

(VI)

wobei

m eine ganze Zahl von 0 bis 20 ist,
o eine ganze Zahl von 0 bis 20 ist,
p eine ganze Zahl von 1 bis 40 ist,
$R^1$ ein Wasserstoffatom oder eine lineare $C_{1-6}$-, verzweigte $C_{2-6}$- oder cyclische $C_{3-6}$-Alkylgruppe darstellt,
$R^3$ eine lineare $C_{1-6}$- oder verzweigte oder cyclische $C_{3-6}$-Alkylgruppe darstellt,
$R^4$ eine Vinylgruppe, eine Vinylethergruppe, eine Vinylestergruppe, eine Aziridingruppe, eine Oxetangruppe darstellt oder eine lineare $C_{1-6}$- oder eine verzweigte oder cyclische $C_{3-6}$-Alkylgruppe darstellt, und

wobei n eine mittlere Kettenlänge im Bereich von 1 bis 50 ist.

**9.** Verwendung eines polymerisierbaren Makromonomers der Formel (VI) wie in Anspruch 8 definiert in einer dentalen Zusammensetzung.

**10.** Verwendung nach Anspruch 9, wobei die dentale Zusammensetzung ein dentales Abformmaterial, ein fließfähiges Dentalkomposit, ein universelles Dentalkomposit, ein stopfbares Dentalkomposit oder ein Fissurenversiegler ist.

**Revendications**

**1.** Matériau d'empreinte dentaire comprenant

(i) une composition polymérisable contenant un composé ayant des unités récurrentes de la formule (I) suivante :

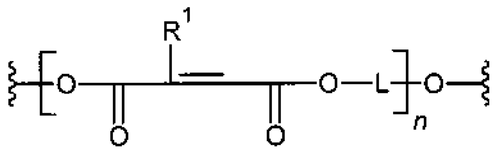

(I)

où

$R^1$ représente un atome d'hydrogène ou un groupe alkyle en $C_{1-6}$ linéaire, ou un groupe alkyle en $C_{3-6}$ ramifié ou cyclique ;
L représente un groupe de liaison organique divalent qui contient un ou plusieurs groupes organopolysiloxane monovalents ou divalents et/ou un ou plusieurs groupes perfluorohydrocarbyle monovalents ou divalents ; et
n est un entier supérieur ou égal à 1 ;

(ii) un ou plusieurs composés copolymérisables avec le composé ayant des unités récurrentes de formule (I) ; et
(iii) un système initiateur de polymérisation.

**2.** Matériau d'empreinte dentaire selon la revendication 1, dans lequel la composition polymérisable contenant un composé ayant des unités récurrentes de formule (I) peut être obtenue en faisant réagir un mélange comprenant :

(a) x équivalents d'un ou plusieurs composés de la formule (II) suivante :

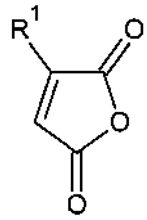

(II)

où

$R^1$ est tel que défini dans la revendication 1 ;

(b) y équivalents d'un composé de la formule (III) suivante :

HO-L-OH (III)

où

L est tel que défini dans la revendication 1 ; et

(c) éventuellement z équivalents d'un ou plusieurs composés de la formule (IV) ou (V) suivante :

$R^2$-COOH (IV)

$R^2$-OH (V)

où

$R^2$ représente indépendamment un groupe organique pouvant contenir un ou plusieurs groupes organopolysiloxane monovalents ou divalents et/ou un ou plusieurs groupes perfluorohydrocarbyle monovalents ou divalents, et/ou un ou plusieurs groupes polymérisables choisis parmi les groupes (méth)acryloyle, les groupes (méth)acrylamido, les groupes éther vinylique, les groupes ester vinylique, l'aziridine, les groupes époxyde et les groupes oxétane,

où $0,05 \leq x/y \leq 0,66$, et $2y - 2x \leq z \leq 1,5(2y - 2x)$, où x, y et z sont les équivalents molaires des composants (a), (b) et (c).

3. Matériau d'empreinte dentaire selon la revendication 1 ou 2, dans lequel $R^1$ est un atome d'hydrogène.

4. Matériau d'empreinte dentaire selon l'une quelconque des revendications précédentes, dans lequel L est un groupe de liaison organique divalent contenant un groupe organopolysiloxane divalent.

5. Matériau d'empreinte dentaire selon l'une quelconque des revendications précédentes, dans lequel le groupe organopolysiloxane contient un groupe polymérisable choisi parmi les groupes éther vinylique, ester vinylique, oxétane et aziridine.

6. Matériau d'empreinte dentaire selon l'une quelconque des revendications précédentes, qui est adapté pour former un réseau interpénétrant (IPN) par une combinaison d'une polymérisation radicalaire libre et d'une polymérisation cationique.

7. Matériau d'empreinte dentaire selon l'une quelconque des revendications précédentes, dans lequel la composition polymérisable (i) a un indice de réfraction compris entre 1,450 et 1,540.

8. Composition polymérisable comprenant des macromonomères polymérisables d'une de la formule (VI) suivante :

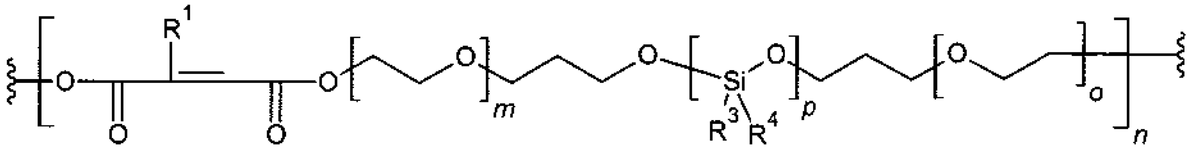

(VI)

où

m est un entier de 0 à 20,
o est un entier de 0 à 20,
p est un entier de 1 à 40,
$R^1$ représente un atome d'hydrogène ou un groupe alkyle en $C_{1-6}$ linéaire, en $C_{2-6}$ ramifié ou en $C_{3-6}$ cyclique,
$R^3$ représente un groupe alkyle en $C_{1-6}$ linéaire ou en $C_{3-6}$ ramifié ou cyclique,
$R^4$ représente un groupe vinylique, un groupe éther vinylique, un groupe ester vinylique, un groupe aziridine, un groupe oxétane ou représente un groupe alkyle en $C_{1-6}$ linéaire, ou en $C_{3-6}$ ramifié ou cyclique, et

dans laquelle n est une longueur de chaîne moyenne comprise entre 1 et 50.

**9.** Utilisation d'un macromonomère polymérisable de la formule (VI) tel que défini dans la revendication 8 dans une composition dentaire.

**10.** Utilisation selon la revendication 9, dans laquelle la composition dentaire est un matériau d'empreinte dentaire, un composite dentaire fluide, un composite dentaire universel, un composite dentaire compactable ou un scellant de cavités et fissures.

Fig. 1

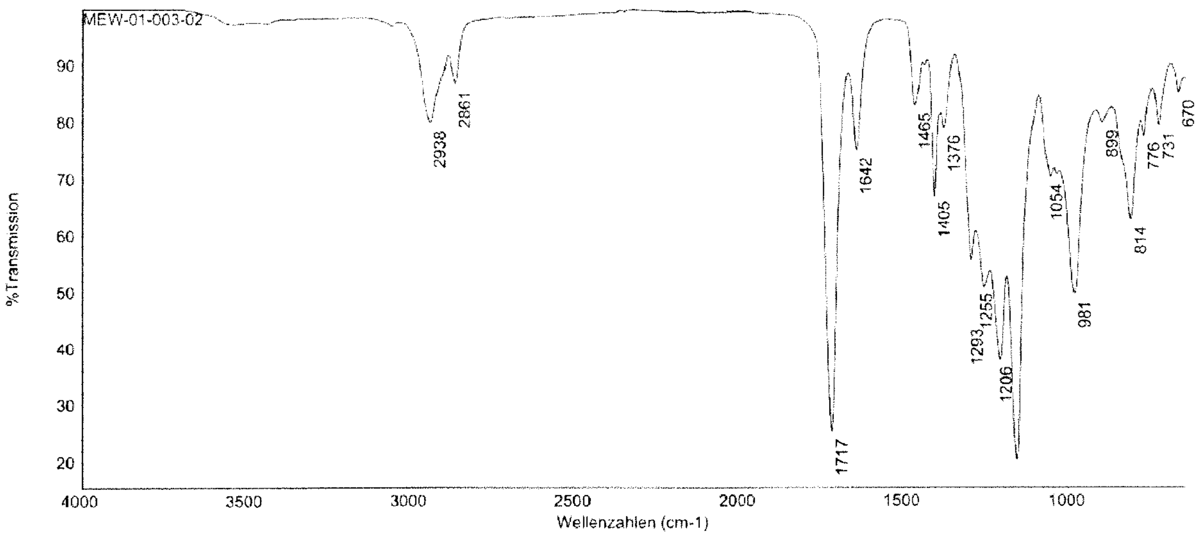
MEW-01-003-02
%Transmission
90
80
70
60
50
40
30
20
2938
2861
1717
1642
1465
1405
1376
1293
1255
1206
1054
981
899
814
776
731
670
4000
3500
3000
2500
2000
1500
1000
Wellenzahlen (cm-1)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 102010046697 A1
- US 5545676 A
- US 4298738 A
- US 4324744 A
- US 4385109 A
- EP 0173567 A


### Non-patent literature cited in the description


- **K. AALTO-KORTE et al.** *Contact Dermatitis,* December 2007, vol. 57 (5), 324-30
- **YAMAMOTO K. et al.** *J. Tetrahedron Lett.,* 1980, vol. 21, 1653-1656
- **NICEWICZ D.A. et al.** *Org. Synth.,* 2008, vol. 85, 278-286
- **NICEWICZ D.A.** *J. Am. Chem. Soc.,* 2005, vol. 127 (17), 6170-6171
- **BOYCE G.R. et al.** *J. Org. Chem.,* 2012, vol. 77 (10), 4503-4515
- **BOYCE G.R. et al.** *Org. Lett.,* 2012, vol. 14 (2), 652-655
- *CHEMICAL ABSTRACTS,* 1171-49-9
- **El-ROZ, M. et al.** *Current Trends in Polymer Science,* 2011, vol. 15, 1-13
- **KRICHELDORF, H. R. ; YASHIRO, T. ; WEIDNER, S.** *Macromolecules,* 2009, vol. 42, 6433-6439